# EUROPÄISCHE PATENTSCHRIFT

(11) **EP 0 755 915 B1**
(45) Veröffentlichungstag und Bekanntmachung des Hinweises auf die Patenterteilung: **06.09.2000**
(21) Anmeldenummer: 96111571.4
(22) Anmeldetag: 18.07.1996
(51) Int. Cl.: C07C 69/92, C09K 19/58, C07C 69/94, C07D 239/34, C07C 69/753

(54) **Photovernetzbare Chirale Dotierstoffe**
Photocrosslinkable chiral doping agents
Agents de dopage chiraux photoréticulables

(30) Priorität: 28.07.1995 CH 222095; 09.05.1996 EP 96107333
(43) Veröffentlichungstag der Anmeldung: 29.01.1997
(73) Patentinhaber: Rolic AG, 6301 Zug (CH)
(72) Erfinder: Kelly, Stephen, Beverley, East Yorkshire HU17 8XA (GB)
(74) Vertreter: England, Christopher David

(56) Entgegenhaltungen:
- WO-A-95/16007
- DE-A- 3 822 121

## Beschreibung

Die vorliegende Erfindung betrifft photovernetzbare, optisch aktive Verbindungen, flüssigkristalline Gemische, die solche Verbindungen enthalten, sowie deren Verwendung im vernetzten Zustand als optische Bauelemente.

Durch geeignete Orientierungsschichten oder in einem Feld können photovernetzbare Flüssigkristalle, welche mit einer geeigneten Menge eines Photoinitiators versehen sind, auf einem Substrat oder in einer Zelle orientiert werden und dann in diesem Zustand durch Bestrahlen mit Licht einer geeigneten Wellenlänge vernetzt werden. Die dadurch erzeugte Struktur bleibt auch bei hohen Temperaturen erhalten. So lassen sich optische Bauelemente, wie zum Beispiel Wellenleiter, optische Gitter und Filter, piezoelektrische Zellen und Zellen mit nicht-linearen optischen (NLO) Eigenschaften, elektrooptische Vorrichtungen, usw. herstellen. Solche optische Bauelemente können zum Beispiel in Zellen für Frequenzverdoppelung (SHG) oder in Farbfilter verwendet werden.

Weitere Eigenschaften, wie beispielsweise die Doppelbrechung, der Brechungsindex, die Transparenz, usw., müssen je nach Anwendungsgebiet unterschiedlichen Anforderungen genügen. Beispielsweise sollten Materialien für Farbfilter, Polarisatoren, λ/2 Platten, usw., für zirkular polarisiertes Licht eine kleine Ganghöhe erzeugen.

Die photovernetzbaren Flüssigkristalle müssen eine gute chemische und thermische Stabilität, gute Löslichkeit in gängigen Lösungsmitteln und eine gute Stabilität gegenüber elektrischen Feldern und elektromagnetischer Strahlung aufweisen. Sie sollten in einem Temperaturbereich von etwa 25°C bis etwa +100°C, insbesondere von etwa 25°C bis etwa +80°C, eine geeignete Mesophase besitzen.

Chiral nematische (cholesterische) Flüssigkristalle im homogen orientierten Zustand (Grandjean Textur) reflektieren Licht im wesentlichen nur in einem Wellenlängebereich, für den die Wellenlänge von der Helixganghöhe abhängig ist. Die spektrale Breite dieses Reflektionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren, usw., ausgenutzt werden. Wenn solche cholesterische Mischungen photochemisch oligomerisierbare oder polymerisierbare Komponenten enthalten, dann können diese Komponenten durch Bestrahlung mit Licht vernetzt werden. Die flüssigkristalline Struktur dieser Mischungen wird dadurch stabilisiert oder sogar eingefroren. So lassen sich Farbfilter, λ/2 Platten, optische Retarder, Polarisatoren, usw., im festem Zustand herstellen.

Da Flüssigkristalle in der Regel als Mischungen mehrerer Komponenten zur Anwendung gelangen, ist es wichtig, dass die Komponenten untereinander gut mischbar sind. Mischungen bestehend aus photovernetzbaren Flüssigkristallen und nicht photovernetzbaren Komponenten, welche in Mischungen zu optischer Aktivität führen, erlauben eine Relaxierung der nicht vernetzten Bestandteile und mindern dadurch die Stabilität des Netzwerkes. Herkömmliche optisch aktive photochemisch oligomerisierbare oder polymerisierbare Zusätze besitzen das Chiralitätszentrum in den Spacern zwischen der photovernetzbaren Gruppe und dem molekularen Kern. Dies erfordert eine aufwendige und komplizierte Synthese. Das Verdrillungsvermögen dieser Verbindungen ist relativ klein.

Es stellte sich somit die Aufgabe, insbesondere für die Anwendung in optischen Filtern, photochemisch oligomerisierbare oder polymerisierbare, optisch aktive Verbindungen herzustellen, welche ein grosses Verdrillungsvermögen besitzen. Damit können sie flüssigkristallinen Mischungen in kleinen Konzentrationen beigemischt werden, um die erwünschte Ganghöhe zu erzeugen, ohne den Klärpunkt oder andere physikalische Eigenschaften der Mischung stark zu beinträchtigen. Dazu sollten sie aus bekannten oder im Handel erhältlichen Ausgangsmaterialien leicht zugänglich sein. Weiter sollten Mischungen, welche solche optisch aktiven photovernetzbaren Verbindungen enthalten, möglichst domänenfrei orientierbar und strukturierbar sein und auch eine ausgezeichnete thermische Stabilität und Langzeitstabilität im vernetzten Zustand aufweisen.

Die vorliegende Erfindung stellt nun Verbindungen, die in hervorragender Weise als Einzelkomponenten oder als Komponenten solcher Flüssigkristall-Mischungen geeignet sind, zur Verfügung. Gegenstand der vorliegenden Erfindung sind Verbindungen der allgemeinen Formel I 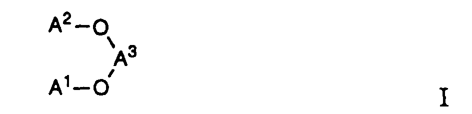 worin
- A¹ und A²: je einen vernetzbaren, mesogenen Rest; und
- A³: (R,R)- bzw. (S,S)-trans-1,2-cyclohexyl-diyl bedeuten.

Da die erfindungsgemässen Verbindungen der Formel I oder Mischungen enthaltend solche Verbindungen eine Mesophase aufweisen, können sie vor dem Vernetzen auf einer Orientierungsschicht durch Anlegen eines elektrischen oder magnetischen Feldes orientiert werden. Dabei wird eine einheitliche Schicht erzeugt.

Vorzugsweise bedeuten die mesogen Reste A¹ und A² je einen Rest der allgemeinen Formel II 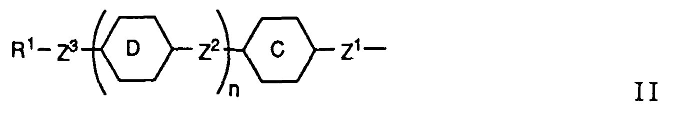 worin
- Ringe C und D: unabhängig voneinander Pyridin-2,5-diyl, Pyrimidin-2,5-diyl, trans-1,4-Cyclohexylen, trans-1,3-Dioxan-2,5-diyl oder gegebenenfalls mit Halogen, Methyl und/oder Cyano substituiertes 1,4-Phenylen;
- Z¹: -(CH₂)ₘ-, -CO-, -(CH₂)ₘCO-, -(CH₂)ₘOOC-;
- Z²: eine Einfachbindung, -CH₂CH₂-, -CH₂O-, -OCH₂-, -COO-, -OOC-, -(CH₂)₄-, -O(CH₂)₃- oder -(CH₂)₃O-;
- Z³: -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂l]H₂O-, oder -NHCH₂(Si[(CH₃)₂]O)ₘSi[(CH₃)₂]CH₂NH-;
- Y: Wasserstoff oder Fluor;
- n: 0, 1 oder 2;
- m: eine ganze Zahl von 1 bis 16; und
- R¹: vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 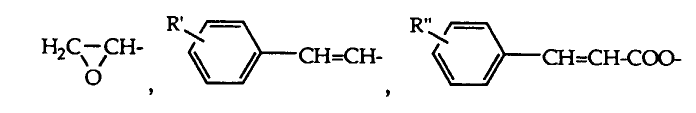
- Ph: Phenyl;
- R': Alkyl mit 1-4 C-Atomen;
- R": Methyl, Methoxy, Cyano oder Halogen bedeuten;
mit der Massgabe, dass R¹-Z³ keine -O-O- oder -N-O- Gruppen enthalten.

Bevorzugt werden Verbindungen der Formel I, worin die beiden mesogenen Reste A¹ und A² die gleiche Bedeutung haben.

Besonders bevorzugt werden Reste A¹ und A² der Formel II, worin die Ringe C und D unabhängig voneinander unsubstituiertes oder mit Fluor substituiertes 1,4-Phenylen, Pyridin-2,5-diyl, Pyrimidin-2,5-diyl oder trans-1,4-Cyclohexylen; Z¹ -CH₂-, -CO- oder -OOC-; Z² eine Einfachbindung, -CH₂CH₂-, -CH₂O-, OCH₂-, -COO- oder -OOC-; Z³ -(CH₂)ₘ-, -(CH₂)ₘO-, -(CH₂)ₘCOO- oder -(CH₂)ₘOOC- bedeuten.

Vorzugsweise bedeutet die vernetzbare Gruppe R¹ CH₂=CH-COO-, CH₂=C(CH₃)-COO-, CH₂=C(Cl)-COO-, CH₂=C(Ph)-COO-, CH₂=CH-COO-Ph-CH₂=CH-CONH-, CH₂=C(CH₃)-CONH-, CH₂=C(Ph)-CONH-, CH₂=CH-O-, CH₂=CH-OOC-, -cis, trans -HOO-CR'=CR'-COO-, 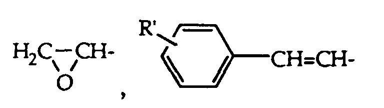 worin R' und R" die oben angegebene Bedeutung haben.

Es sind dies Reste, welche nach Orientieren der Verbindungen der Formel I in einem Feld photochemisch vernetzt werden können.

Besonders bevorzugte Gruppen R¹ sind CH₂=CH-COO-, CH₂=C(CH₃)-COO-, CH₂=CH-O-,

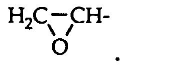

Die oben verwendeten Ausdrücke werden im folgenden erläutert:

"Gegebenenfalls mit Halogen, Methyl und/oder Cyano substituiertes 1,4-Phenylen" umfasst in der vorliegenden Erfindung 1,4-Phenylen, mit Fluor, Brom, Chlor, Methyl oder Cyano ein- oder mehrfach substituiertes 1,4-Phenylen wie beispielsweise 2- bzw. 3-Fluor-1,4-phenylen, 2,3-Difluor-1,4-phenylen, 2,6- bzw. 3,5-Difluor-1,4-phenylen, 2- bzw. 3-Chlor-1,4-phenylen, 2,3-Dichlor-1,4-phenylen, 2,6- bzw. 3,5-Dichlor-1,4-phenylen, 2- bzw. 3-Brom-1,4-phenylen, 2- bzw. 3-Methyl-1,4-phenylen, 2- bzw. 3-Cyano-1,4-phenylen und dergleichen.

"Halogen" bedeutet Fluor, Chlor oder Brom, insbesondere Fluor.

"Niederes Alkyl" bedeutet eine geradkettige oder verzweigte Alkylgruppe mit 1 bis 4 Kohlenstoffatomen, wie beispielsweise Methyl, Ethyl, Propyl, Butyl, i-Propyl, i-Butyl, tert.-Butyl, insbesondere jedoch Methyl, Ethyl, Propyl oder Butyl.

Der Mesophasen-Typ der erfindungsgemässen Verbindungen kann durch Variation der Ringe in den Seitenketten A¹ und A² beeinflusst werden. So haben aromatische Ringe wie Phenylen die Tendenz smektische Phasen zu erzeugen, während gesättigte Ringe wie trans-1,4-Cyclohexylen oder trans-1,3-Dioxan-2,5-diyl Ringe nematische Tendenzen fördern.

Vorzugsweise bedeuten die mesogenen Reste A¹ und A² einen Rest der Formel II, worin n = 1 ist, das heisst einen Rest der Formel II-a 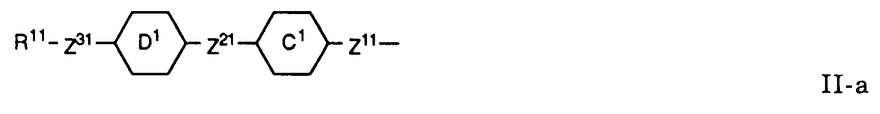 worin
- Ringe C¹ und D¹: unabhängig voneinander Pyridin-2,5-diyl, Pyrimidin-2,5-diyl oder gegebenenfalls mit Fluor substituiertes 1,4-Phenylen, trans-1,4-Cyclohexylen;
- Z¹¹: -CH₂- oder -CO-;
- Z²¹: eine Einfachbindung, -CH₂O-, -COO-, -OOC-;
- Z³¹: -(CH₂)_{m'}-, -(CH₂)_{m'}O-, -(CH₂)_{m'}COO- oder -(CH₂)_{m'}OOC-;
- m': eine ganze Zahl von 3 bis 12; und
- R¹¹: CH₂=CH-COO-, CH₂=C(CH₃)-COO-, CH₂=CH-O-, 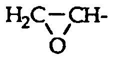
bedeuten.

Besonders bevorzugt werden Verbindungen der Formeln 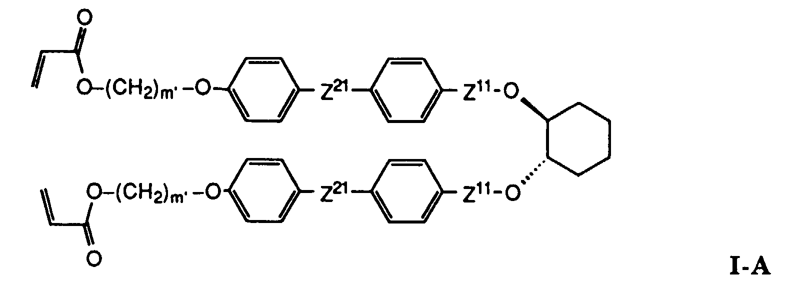 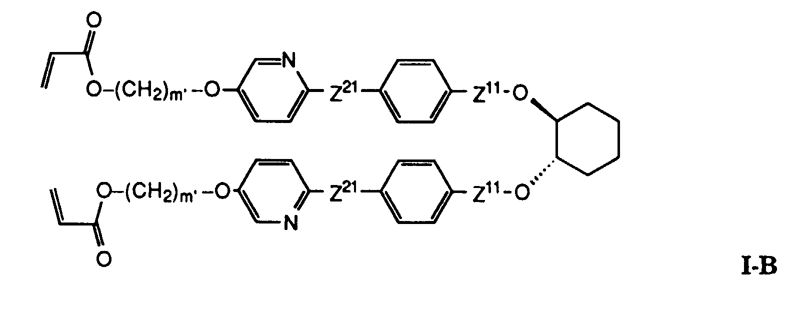 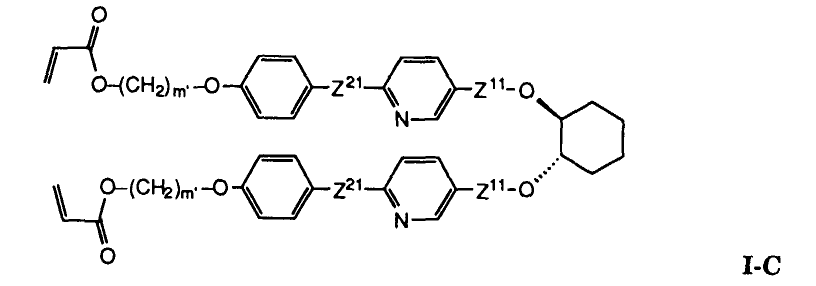 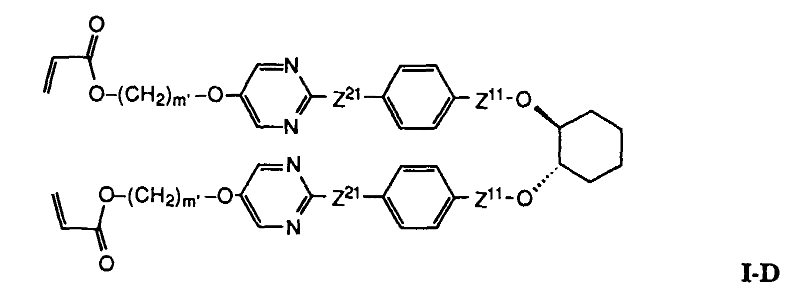 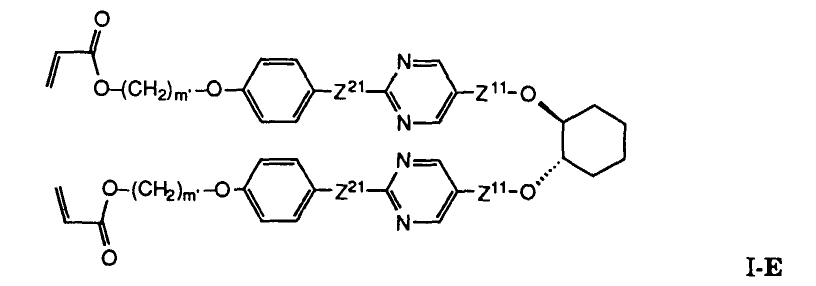 worin Z¹¹ -CO- und Z²¹ eine Einfachbindung oder -COO-, insbesondere jedoch -COO-, bedeuten, und m' eine ganze Zahl von 3 bis 12 ist, sowie die Enantiomeren dieser Verbindungen.

Die Verbindungen der Formel I, worin A¹ und A² die gleiche Bedeutung haben, sind synthetisch sehr einfach zugänglich und können beispielsweise analog zu den in den Schemata 1 bis 5 aufgezeigten Methoden hergestellt werden. So können in an sich bekannter Weise optisch aktive Diole mit (ω-acryloyloxyalkyloxy)-substituierten Carbonsäuren umgesetzt werden. Diese Veresterung kann beispielsweise über das entsprechende Methylsulfonat in Tetrahydrofuran oder in Gegenwart von N,N'-Dicyclohexylcarbodiimid und 4-(Dimethylamino)pyridin in Dichlormethan oder einem anderen geeigneten Lösungsmittel wie z.B. Chloroform erfolgen. Optisch aktive Diole können auch in einer Williamson Verätherung mit (ω-acryloyloxyalkyloxy)-substituierten Benzyltosylaten umgesetzt werden. Diese Veretherung kann beispielsweise bei Raumtemperatur in Gegenwart von Kalium-tert.-butylat in Dimethoxyethan oder einem anderen geeigneten Lösungsmittel, wie z.B. N, N'-Dimethylfomamid, erfolgen.

Verbindungen der Formel I, worin A¹ und A² verschieden sind, können durch Monoveresterung von optisch aktiven Diolen mit einer (ω-acryloyloxyalkyloxy)-substituierten Carbonsäure und anschliessende Veresterung mit einer anderen (ω-acryloyloxyalkyloxy)-substituierten Carbonsäure hergestellt werden. Die entsprechenden asymmetrischen Diether sind über ein ähnliches zweistufiges Verfahren zugänglich (beschrieben). Die Ausgangsmaterialien sind bekannt und z.T. im Handel erhältlich.

Die in den Schemata 1 bis 5 aufgeführten Reaktionen erfolgen alle in an sich bekannter Weise. In den Schemata haben die Symbole die obengenannten Bedeutungen.

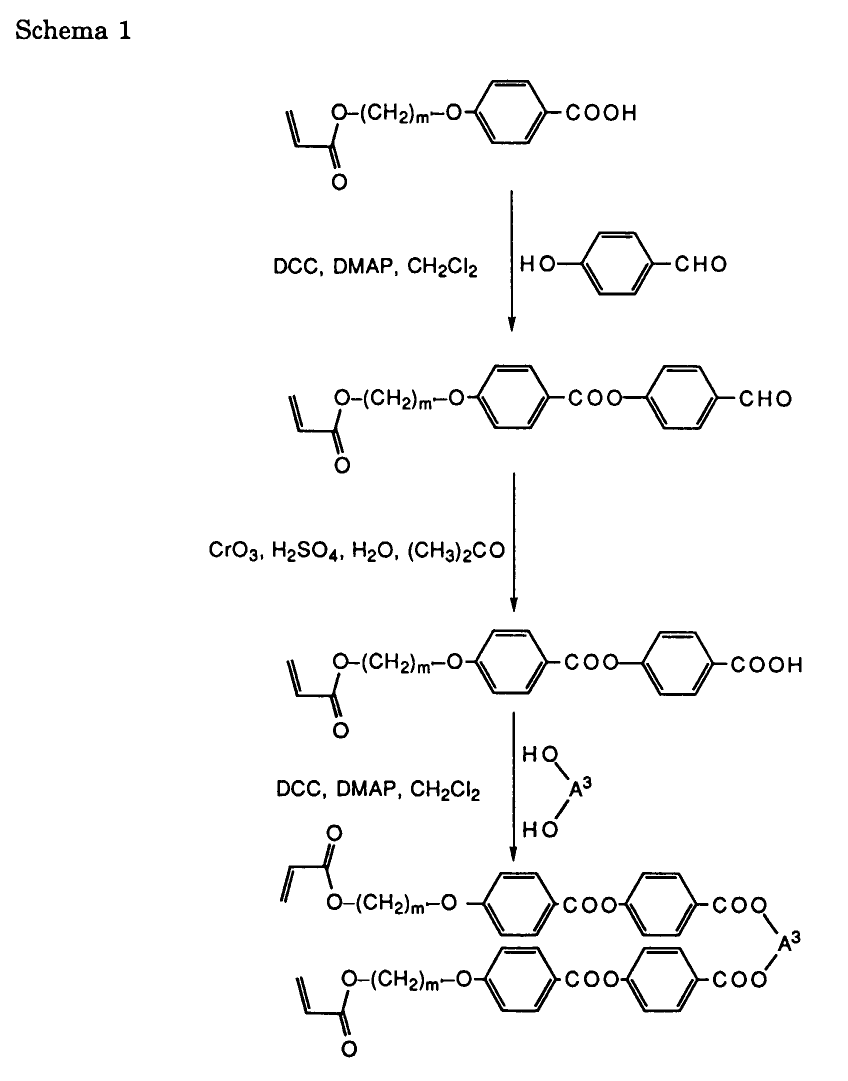

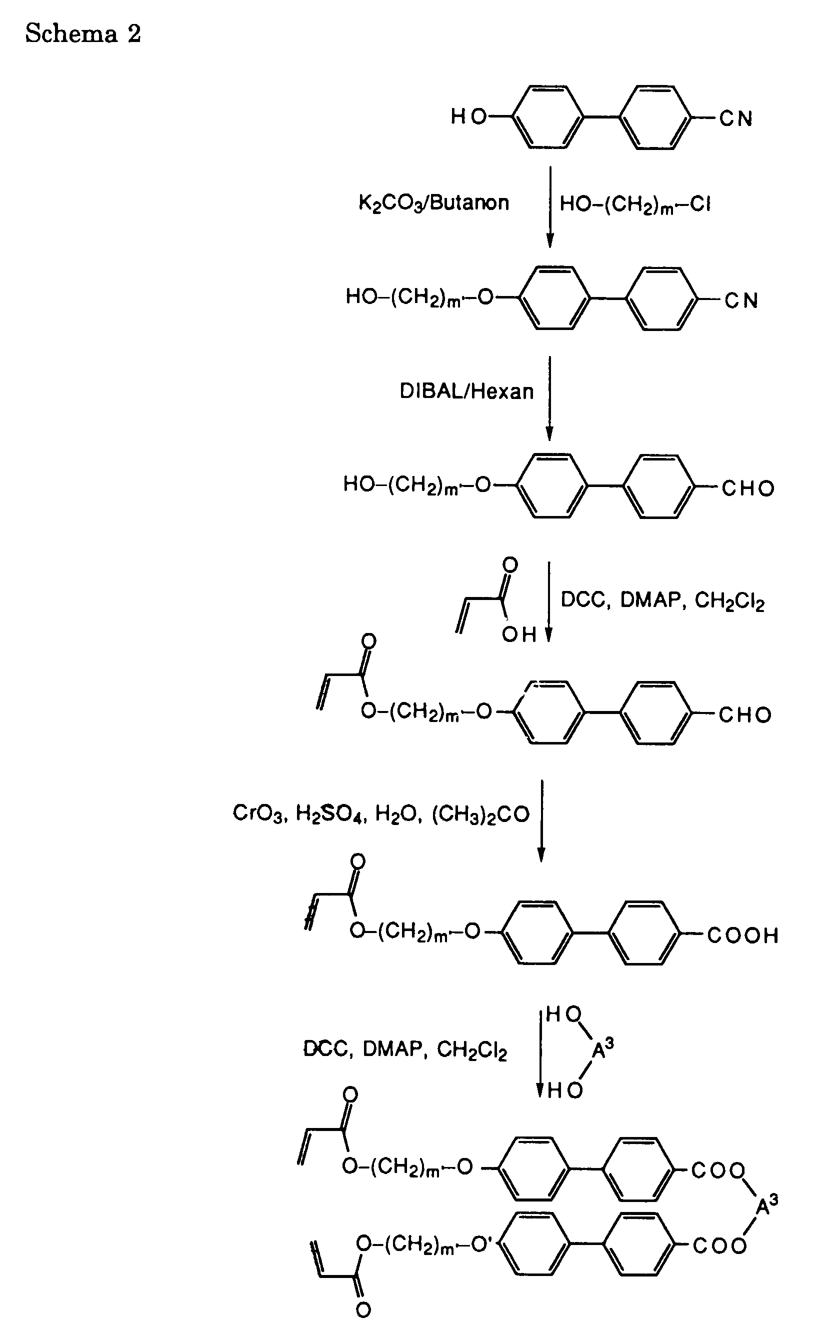

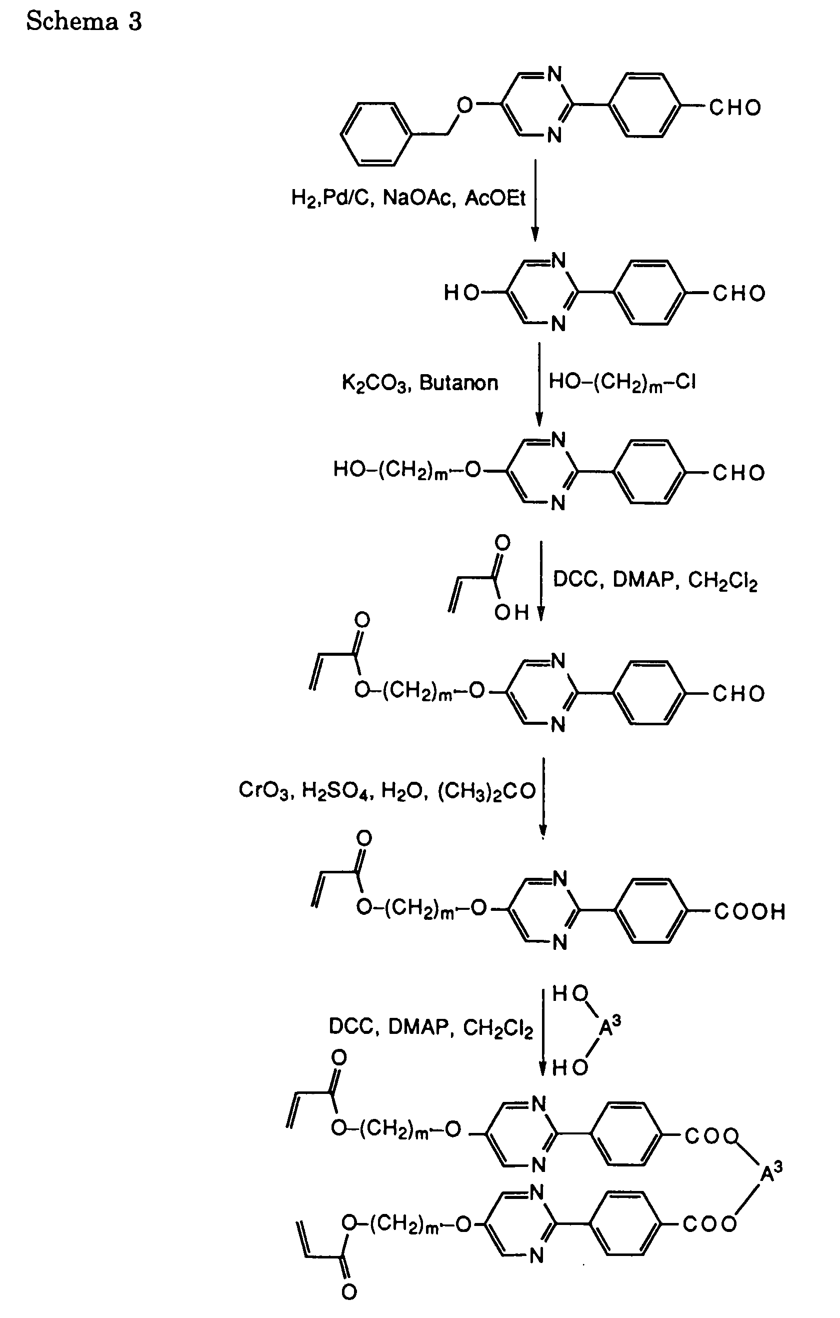

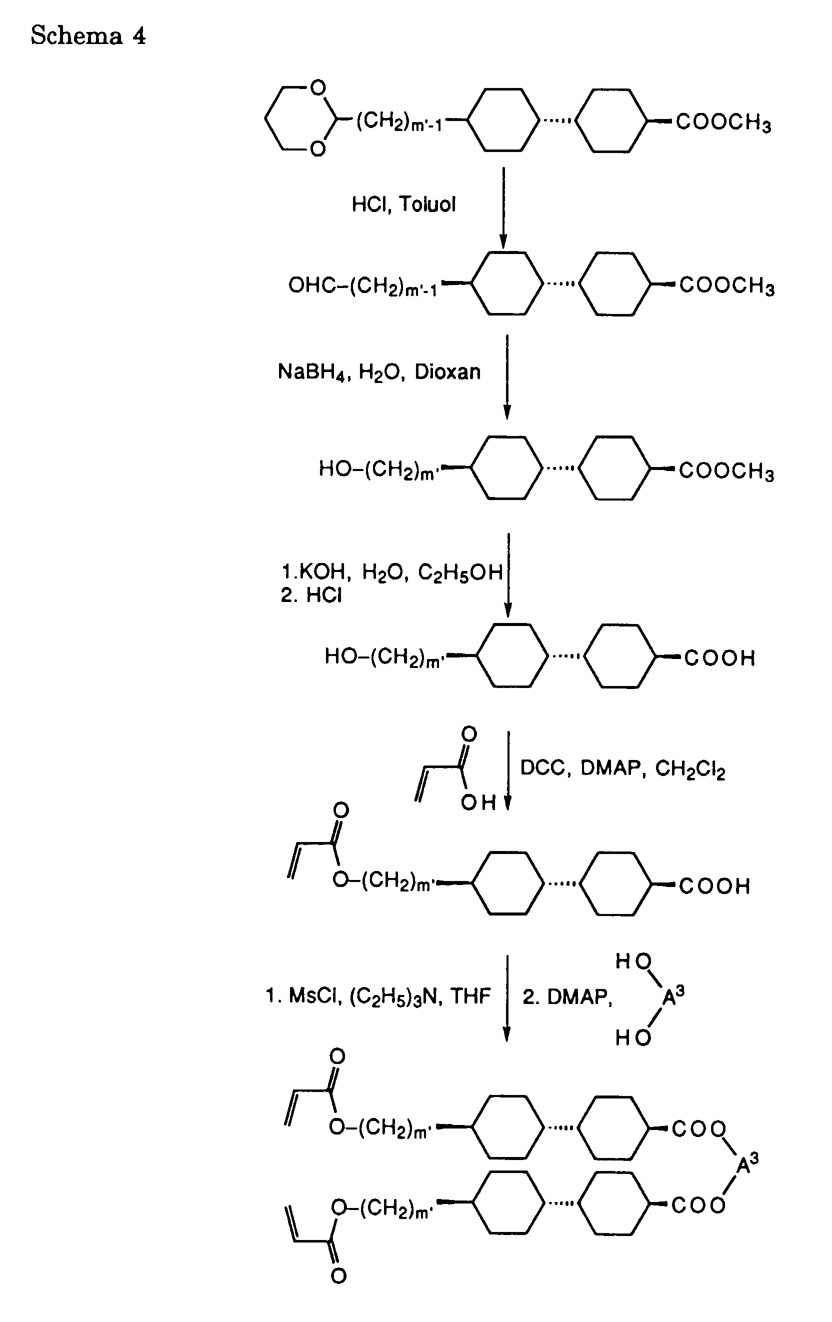

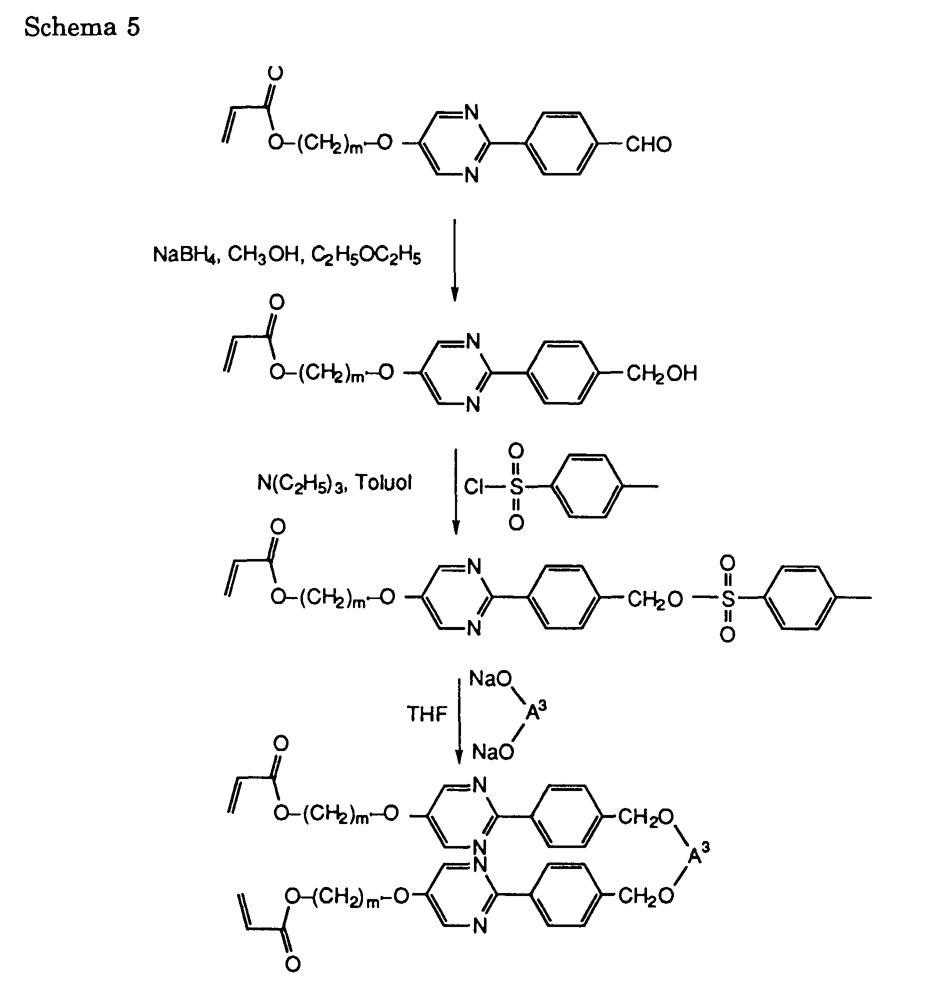

Eine kleine Menge BHT (2,6-di-tert.-Butyl-4-methyl-phenol/"Butylhydroxytoluol") wird jeder Stufe beigemischt, um unerwünschtes thermisches Vernetzen zu unterbinden.

Die Verbindungen der Formel I werden als chirale Dotierstoffe in flüssigkristallinen Gemischen verwendet. Diese flüssigkristallinen Gemische enthalten mindestens eine Verbindung der Formel I.

In den erfindungsgemässen flüssigkristallinen Gemischen kann der Anteil an chiralen Verbindungen der Formel I in einem breiten Bereich variieren und beispielweise etwa 0, 1-30 Gew. % betragen und wird im wesentlichen durch das Verdrillungsvermögen der Verbindungen und die gewünschte Ganghöhe bestimmt.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder andere, bekannte flüssigkristalline Verbindungen mit einer photovernetzbaren Gruppe sein. Es können auch noch weitere chirale Komponenten im Gemisch enthalten sein.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 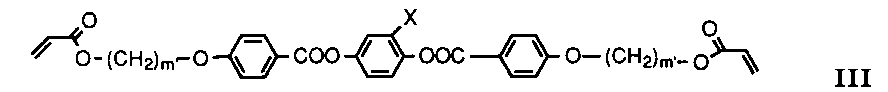 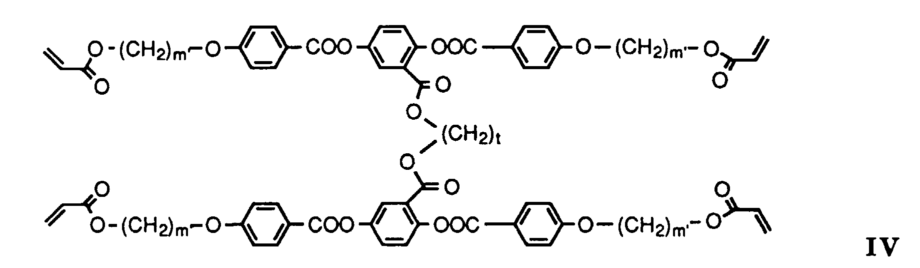 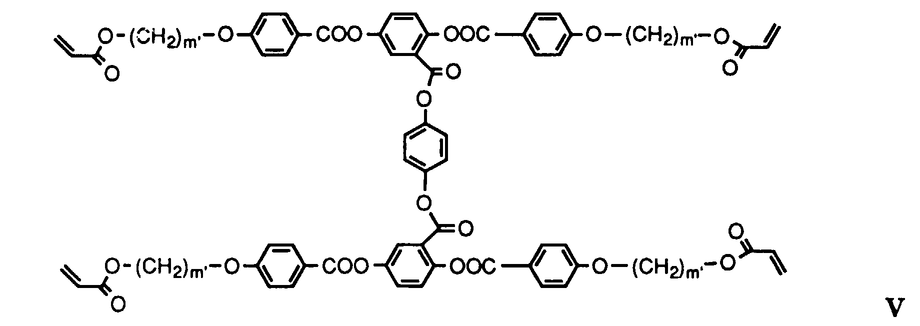 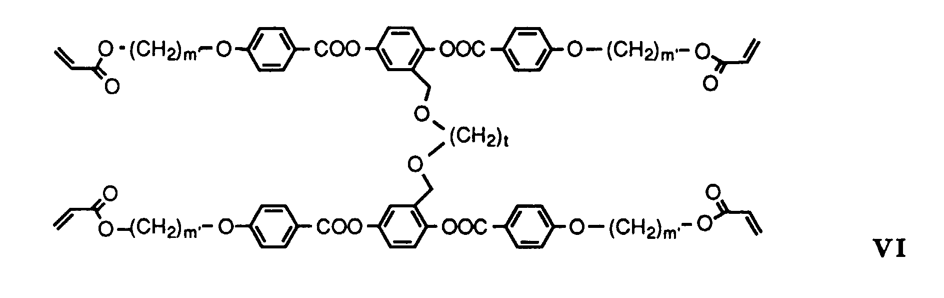 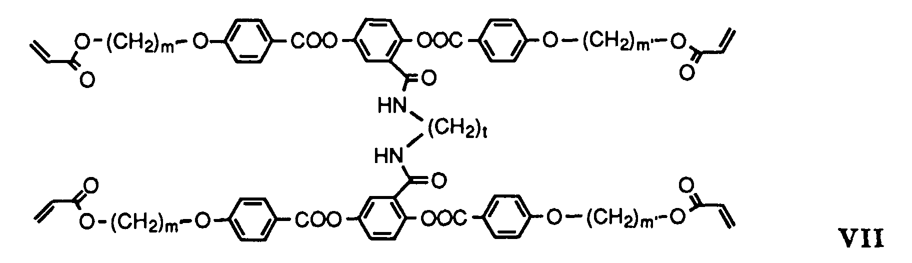 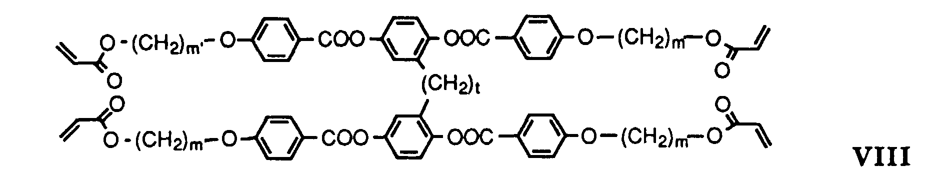 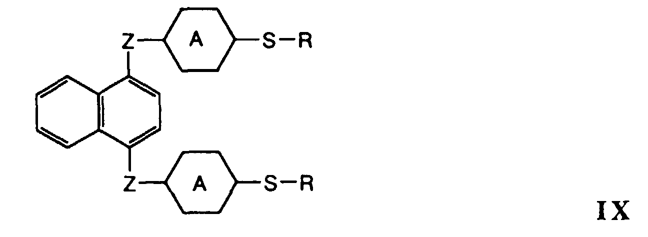 und 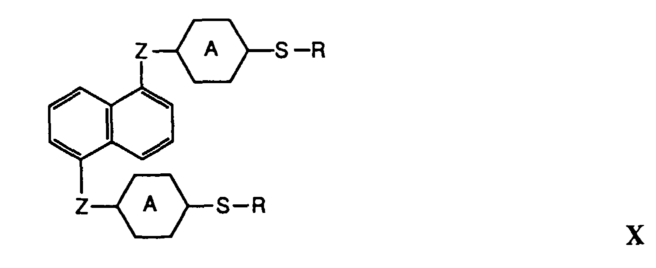 worin
- X: Wasserstoff, Fluor, Chlor, Brom oder Methyl;
- m': eine ganze Zahl von 4 bis 12;
- t: eine ganze Zahl von 2 bis 12 ist;
- Z: -OCH₂- oder -OOC-;
- A: 1,4-Phenylen oder 2- bzw. 3-Fluor-1,4-phenylen;
- S: -(CH₂)_{m'}-,-(CH₂)_{m'}O- oder-O(CH₂)_{m'}-; und
- R: CH₂=CH-COO-, CH₂=C(CH₃)-COO-, CH₂=CH-O-, 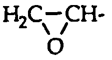
bedeuten.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N eine nematische und I die isotrope Phase. Optische Antipoden besitzen jeweils "spiegelbildliche Eigenschaften", d.h. gleiche Schmelzpunkte usw., führen aber zu entgegengesetztem Helixdrehsinn und entgegengesetzter Zirkularpolarisation des reflektierten Lichtes.

### Beispiel 1

Zu einer Lösung von 0,1 g (1R,2R)-trans-1,2-Cyclohexandiol, 0,9 g 4-(4-[8-Acryloyloxyoctyloxy]phenylcarbonyloxy)benzoesäure und 0,05 g 4-(Dimethylamino)pyridin in 20 ml Dichlormethan wurde unter Rühren innert 5 Minuten 0,44 g N,N '-Dicyclohexylcarbodiimid gegeben. Das Reaktionsgemisch wurde über Nacht gerührt, filtriert und das Filtrat eingeengt. Chromatographie des Rückstandes an Kieselgel mit Hexan/ Aethylacetat (Vol. 8:2) und Umkristallisation der gemäss Dünnschichtchromatographie reinen Fraktionen aus Ethylalkohol ergab 0,1 g (1R,2R)-trans-1,2-bis[4-(4-[8-Acryloyloxyoctyloxy]phenylcarbonyloxy)phenylcarbonyloxy]cyclohexan; Smp. (C-I) 97°C.

In analoger Weise können folgende Verbindungen hergestellt werden:

(1R,2R)-trans-1,2-bis[4-(5-[4-Acryloyloxybutyloxy]pyrimidin-2-yl)phenylcarbonyloxy]cyclohexan; Smp. (C-I) 107°C.

(1R,2R)-trans-1,2-bis[2-(4-[4-Acryloyloxybutyloxy]phenyl)pyridin-5-yl)carbonyloxy]cyclohexan; Smp. (C-I) 108°C.

(1R,2R)-trans-1,2-bis[trans-4-(4-[6-Acryloyloxyhexyloxy]phenyl)cyclohexyl-ethylcarbonyloxy]cyclohexan; Smp. (C-I) 54 °C.

Ebenso können hergestellt werden:
(1R,2R)-trans-1,2-bis[4-(4-[3-Acryloyloxypropyloxy]phenylcarbonyloxy)phenylcarbonyloxy]cyclohexan;
(1R,2R)-trans-1,2-bis[4-(4-[4-Acryloyloxybutoxy]phenylcarbonyloxy)phenylcarbonyloxy]cyclohexan;
(1R,2R)-trans-1,2-bis[4-(4-[5-Acryloyloxypentyloxy]phenylcarbonyloxy)phenylcarbonyloxy]cyclohexan;
(1R,2R)-trans-1,2-bis[4-(4-[6-Acryloyloxyhexyloxy]phenylcarbonyloxy)phenylcarbonyloxy]cyclohexan;
(1R,2R)-trans-1,2-bis[4-(4-[7-Acryloyloxyheptyloxy]phenylcarbonyloxy)phenylcarbonyloxy]cyclohexan;
(1R,2R)-trans-1,2-bis[4-(4-[9-Acryloyloxynonyloxy]phenylcarbonyloxy)phenylcarbonyloxy]cyclohexan;
(1R,2R)-trans-1,2-bis[4-(4-[10-Acryloyloxydecyloxy]phenylcarbonyloxy)phenylcarbonyloxy]cyclohexan;
(1R,2R)-trans-1,2-bis[4-(4-[11-Acryloyloxyundecyloxy]phenylcarbonyloxy)phenylcarbonyloxy]cyclohexan;
(1R,2R)-trans-1,2-bis[4-(4-[12-Acryloyloxydodecyloxy]phenylcarbonyloxy)phenylcarbonyloxy]cyclohexan;
(1R,2R)-trans-1,2-bis(4-[3-Acryloyloxypropyloxy]biphenyl-4'-carbonyloxy)cyclohexan;
(1R,2R)-trans-1,2-bis(4-[4-Acryloyloxybutoxy]biphenyl-4'-carbonyloxy)cyclohexan;
(1R,2R)-trans-1,2-bis(4-[5-Acryloyloxypentyloxy]biphenyl-4'-carbonyloxy)cyclohexan;
(1R,2R)-trans-1,2-bis(4-[6-Acryloyloxyhexyloxy]biphenyl-4'-carbonyloxy)cyclohexan;
(1R,2R)-trans-1,2-bis(4-[7-Acryloyloxyheptyloxy]biphenyl-4'-carbonyloxy)cyclohexan;
(1R,2R)-trans-1,2-bis(4-[8-Acryloyloxyoctyloxy]biphenyl-4'-carbonyloxy)cyclohexan;
(1R,2R)-trans-1,2-bis(4-[9-Acryloyloxynonyloxy]biphenyl-4'-carbonyloxy)cyclohexan;
(1R,2R)-trans-1,2-bis(4-[10-Acryloyloxydecyloxy]biphenyl-4'-carbonyloxy)cyclohexan;
(1R,2R)-trans-1,2-bis(4-[11-Acryloyloxyundecyloxy]biphenyl-4'-carbonyloxy)cyclohexan;
(1R,2R)-trans-1,2-bis(4-[12-Acryloyloxydodecyloxy]biphenyl-4'-carbonyloxy)cyclohexan;
(1R,2R)-trans-1,2-bis[4-(5-[3-Acryloyloxypropyloxy]pyrimidin-2-yl)phenylcarbonyloxy]cyclohexan;
(1R,2R)-trans-1,2-bis[4-(5-[5-Acryloyloxypentyloxy]pyrimidin-2-yl)phenylcarbonyloxy]cyclohexan;
(1R,2R)-trans-1,2-bis[4-(5-[6-Acryloyloxyhexyloxy]pyrimidin-2-yl)phenylcarbonyloxy]cyclohexan;
(1R,2R)-trans-1,2-bis[4-(5-[7-Acryloyloxyheptyloxy]pyrimidin-2-yl)phenylcarbonyloxy]cyclohexan;
(1R,2R)-trans-1,2-bis[4-(5-[8-Acryloyloxyoctyloxy]pyrimidin-2-yl)phenylcarbonyloxy]cyclohexan;
(1R,2R)-trans-1,2-bis[4-(5-[9-Acryloyloxynonyloxy]pyrimidin-2-yl)phenylcarbonyloxy]cyclohexan;
(1R,2R)-trans-1,2-bis[4-(5-[10-Acryloyloxydecyloxy]pyrimidin-2-yl)phenylcarbonyloxy]cyclohexan;
(1R,2R)-trans-1,2-bis[4-(5-[11-Acryloyloxyundecyloxy]pyrimidin-2-yl)phenylcarbonyloxy]cyclohexan;
(1R,2R)-trans-1,2-bis[4-(5-[12-Acryloyloxydodecyloxy]pyrimidin-2-yl)phenylcarbonyloxy]cyclohexan;
(1R,2R)-trans-1,2-bis[4-(5-[3-Acryloyloxypropyloxy]pyridin-2-yl)phenylcarbonyloxy]cyclohexan;
(1R,2R)-trans-1,2-bis[4-(5-[4-Acryloyloxybutyloxy]pyridin-2-yl)phenylcarbonyloxy]cyclohexan;
(1R,2R)-trans-1,2-bis[4-(5-[5-Acryloyloxypentyloxy]pyridin-2-yl)phenylcarbonyloxy]cyclohexan;
(1R,2R)-trans-1,2-bis[4-(5-[6-Acryloyloxyhexyloxy]pyridin-2-yl)phenylcarbonyloxy]cyclohexan;
(1R,2R)-trans-1,2-bis[4-(5-[7-Acryloyloxyheptyloxy]pyridin-2-yl)phenylcarbonyloxy]cyclohexan;
(1R,2R)-trans-1,2-bis[4-(5-[8-Acryloyloxyoctyloxy]pyridin-2-yl)phenylcarbonyloxy]cyclohexan;
(1R,2R)-trans-1,2-bis[4-(5-[9-Acryloyloxynonyloxy]pyridin-2-yl)phenylcarbonyloxy]cyclohexan;
(1R,2R)-trans-1,2-bis[4-(5-[10-Acryloyloxydecyloxy]pyridin-2-yl)phenylcarbonyloxy]cyclohexan;
(1R,2R)-trans-1,2-bis[4-(5-[11-Acryloyloxyundecyloxy]pyridin-2-yl)phenylcarbonyloxy]cyclohexan;
(1R,2R)-trans-1,2-bis[4-(5-[12-Acryloyloxydodecyloxy]pyridin-2-yl)phenylcarbonyloxy]cyclohexan;
(1R,2R)-trans-1,2-bis[2-(4-[8-Acryloyloxyoctyloxy]phenyl)pyrimidin-5-ylcarbonyloxy]cyclohexan;
(1R,2R)-trans-1,2-bis[2-(4-[8-Acryloyloxyoctyloxy]phenyl)pyridin-5-ylcarbonyloxy]cyclohexan;
(1R,2R)-trans-1,2-bis[trans-4-(trans-4-[3-Acryloyloxypropyl]cyclohexyl)cyclohexancarbonyloxy]cyclohexan;
(1R,2R)-trans-1,2-bis[trans-4-(2-[trans-4-(3-Acryloyloxypropyl)cyclohexyl]ethyl)cyclohexancarbonyloxy]cyclohexan;
(1R,2R)-trans-1,2-bis[4-(trans-4-[3-acryloyloxypropyl]cyclohexyl)phenylcarbonyloxy]cyclohexan;
(1R,2R)-trans-1,2-bis[4-(2-[trans-4-(3-Acryloyloxypropyl)cyclohexyl]ethyl)phenylcarbonyloxy]cyclohexan.

### Beispiel 2

Eine Lösung aus 0,2 g (1R,2R)-trans-1,2-Cyclohexandiol, 0,5 g Natriumhydrid und 50 ml Tetrahydrofuran wird 7 Stunden gerührt, mit 1,1 g 4-(5-[8-Acryloyloxyoctyloxy]pyrimidin-2-yl)benzyltosylat versetzt, das Gemisch über Nacht bei Raumtemperatur weiter gerührt, auf 100 ml Wasser gegossen und dann dreimal mit je 50 ml Diethylether extrahiert. Die organischen Phasen werden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1,1 g (1R,2R)-trans-1,2-bis[4-(5-[8-Acryloyloxyoctyloxy]pyrimidin-2-yl)benzyloxy]cyclohexan;

In analoger Weise können folgende Verbindungen hergestellt werden:
(1R,2R)-trans-1,2-bis[4-(5-[-4-Acryloyloxybutyloxy]phenyl]pyridin-2-yl)methoxy]cyclohexan;
(1R,2R)-trans-1,2-bis[trans-4-(4-[-6-Acryloyloxyhexyloxy]phenyl) cyclohexyl-propoxy]cyclohexan;
(1R,2R)-trans-1,2-bis[4-(4-[5-Acryloyloxypentyloxy]phenylcarbonyloxy)benzyloxy]cyclohexan;
(1R,2R)-trans-1,2-bis[4-(4-[6-Acryloyloxyhexyloxy]phenylcarbonyloxy)benzyloxy]cyclohexan;
(1R,2R)-trans-1,2-bis[4-(4-[7-Acryloyloxyheptyloxy]phenylcarbonyloxy)benzyloxy]cyclohexan;
(1R,2R)-trans-1,2-bis[4-(4-[9-Acryloyloxynonyloxy]phenylcarbonyloxy)benzyloxy]cyclohexan;
(1R,2R)-trans-1,2-bis[4-(4-[10-Acryloyloxydecyloxy]phenylcarbonyloxy)benzyloxy]cyclohexan;
(1R,2R)-trans-1,2-bis[4-(4-[11-Acryloyloxyundecyloxy]phenylcarbonyloxy)benzyloxy]cyclohexan;
(1R,2R)-trans-1,2-bis[4-(4-[12-Acryloyloxydodecyloxy]phenylcarbonyloxy)benzyloxy]cyclohexan;
(1R,2R)-trans-1,2-bis(4-[3-Acryloyloxypropyloxy]biphenyl-4'-methoxy)cyclohexan;
(1R,2R)-trans-1,2-bis(4-[4-Acryloyloxybutoxy]biphenyl-4'-methoxy)cyclohexan;
(1R,2R)-trans-1,2-bis(4-[5-Acryloyloxypentyloxy]biphenyl-4'-methoxy)cyclohexan;
(1R,2R)-trans-1,2-bis(4-[6-Acryloyloxyhexyloxy]biphenyl-4'-methoxy)cyclohexan;
(1R,2R)-trans-1,2-bis(4-[7-Acryloyloxyheptyloxy]biphenyl-4'-methoxy)cyclohexan;
(1R,2R)-trans-1,2-bis(4-[9-Acryloyloxynonyloxy]biphenyl-4'-methoxy)cyclohexan;
(1R,2R)-trans-1,2-bis(4-[10-Acryloyloxydecyloxy]biphenyl-4'-methoxy)cyclohexan;
(1R,2R)-trans-1,2-bis(4-[11-Acryloyloxyundecyloxy]biphenyl-4'-methoxy)cyclohexan;
(1R,2R)-trans-1,2-bis(4-[12-Acryloyloxydodecyloxy]biphenyl-4'-methoxy)cyclohexan;
(1R,2R)-trans-1,2-bis[4-(5-[3-Acryloyloxypropyloxy]pyrimidin-2-yl)benzyloxy]cyclohexan;
(1R,2R)-trans-1,2-bis[4-(5-[4-Acryloyloxybutyloxy]pyrimidin-2-yl)benzyloxy]cyclohexan;
(1R,2R)-trans-1,2-bis[4-(5-[5-Acryloyloxypentyloxy]pyrimidin-2-yl)benzyloxy]cyclohexan;
(1R,2R)-trans-1,2-bis[4-(5-[6-Acryloyloxyhexyloxy]pyrimidin-2-yl)benzyloxy]cyclohexan;
(1R,2R)-trans-1,2-bis[4-(5-[7-Acryloyloxyheptyloxy]pyrimidin-2-yl)benzyloxy]cyclohexan;
(1R,2R)-trans-1,2-bis[4-(5-[8-Acryloyloxyoctyloxy]pyrimidin-2-yl)benzyloxy]cyclohexan;
(1R,2R)-trans-1,2-bis[4-(5-[9-Acryloyloxynonyloxy]pyrimidin-2-yl)benzyloxy]cyclohexan;
(1R,2R)-trans-1,2-bis[4-(5-[10-Acryloyloxydecyloxy]pyrimidin-2-yl)benzyloxy]cyclohexan;
(1R,2R)-trans-1,2-bis[4-(5-[11-Acryloyloxyundecyloxy]pyrimidin-2-yl)benzyloxy]cyclohexan;
(1R,2R)-trans-1,2-bis[4-(5-[12-Acryloyloxydodccyloxy]pyrimidin-2-yl)benzyloxy]cyclohexan;
(1R,2R)-trans-1,2-bis[4-(5-[3-Acryloyloxypropyloxy]pyridin-2-yl)benzyloxy]cyclohexan;
(1R,2R)-trans-1,2-bis[4-(5-[4-Acryloyloxybutyloxy]pyridin-2-yl)benzyloxy]cyclohexan;
(1R,2R)-trans-1,2-bis[4-(5-[5-Acryloyloxypentyloxy]pyridin-2-yl)benzyloxy]cyclohexan;
(1R,2R)-trans-1,2-bis[4-(5-[6-Acryloyloxyhexyloxy]pyridin-2-yl)benzyloxy]cyclohexan;
(1R,2R)-trans-1,2-bis[4-(5-[7-Acryloyloxyheptyloxy]pyridin-2-yl)benzyloxy]cyclohexan;
(1R,2R)-trans-1,2-bis[4-(5-[8-Acryloyloxyoctyloxy]pyridin-2-yl)benzyloxy]cyclohexan;
(1R,2R)-trans-1,2-bis[4-(5-[9-Acryloyloxynonyloxy]pyridin-2-yl)benzyloxy]cyclohexan;
(1R,2R)-trans-1,2-bis[4-(5-[10-Acryloyloxydecyloxy]pyridin-2-yl)benzyloxy]cyclohexan;
(1R,2R)-trans-1,2-bis[4-(5-[11-Acryloyloxyundecyloxy]pyridin-2-yl)benzyloxy]cyclohexan;
(1R,2R)-trans-1,2-bis[4-(5-[12-Acryloyloxydodecyloxy]pyridin-2-yl)benzyloxy]cyclohexan;
(1R,2R)-trans-1,2-bis[(2-[4-(8-Acryloyloxyoctyloxy)phenyl]pyrimidin-5-yl)methoxy]cyclohexan;
(1R,2R)-trans-1,2-bis[(trans-4-(trans-4-[3-Acryloyloxypropyl]cyclohexyl)cyclohexyl)methoxy]cyclohexan;
(1R,2R)-trans-1,2-bis[(trans-4-[2-(trans-4-[3-Acryloyloxypropyl]cyclohexyl)ethyl]cyclohexyl)methoxy]cyclohexan;
(1R,2R)-trans-1,2-bis[4-(trans-4-[3-acryloyloxypropyl]cyclohexyl)benzyloxy]cyclohexan;
(1R,2R)-trans-1,2-bis[4-(2-[trans-4-(3-Acryloyloxypropyl)cyclohexyl]ethyl)benzyloxy]cyclohexan.

## Patentansprüche

1. Verbindungen der allgemeinen Formel I 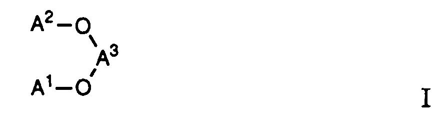 worin
A¹ und A² je einen vernetzbaren, mesogenen Rest; und
A³ (R,R)- bzw. (S,S)-trans-1,2-cyclohexyl-diyl bedeuten.

2. Verbindungen gemäss Anspruch 1, worin die mesogenen Reste A¹ und A² je einen Rest der allgemeinen Formel II 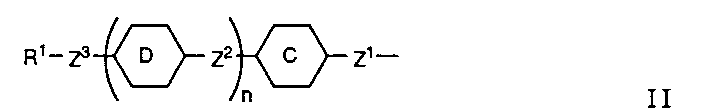 bedeuten, worin
worin
Ringe C und D unabhängig voneinander Pyridin-2,5-diyl, Pyrimidin-2,5-diyl, trans-1,4-Cyclohexylen, trans-1,3-Dioxan-2,5-diyl oder gegebenenfalls mit Halogen, Methyl und/oder Cyano substituiertes 1,4-Phenylen;
Z¹ -(CH₂)ₘ-, -CO-, -(CH₂)ₘCO-, -(CH₂)ₘOOC-;
Z² eine Einfachbindung, -CH₂CH₂-, -CH₂O-, -OCH₂-, -COO-, -OOC-, -(CH₂)₄-, -O(CH₂)₃- oder -(CH₂)₃O-;
Z³ -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, oder -NHCH₂(Si[(CH₃)₂]O)ₘSi[(CH₃)₂]CH₂NH-;
Y Wasserstoff oder Fluor;
n 0, 1 oder 2;
m eine ganze Zahl von 1 bis 16; und
R¹ vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CONH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 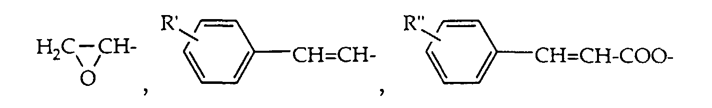
Ph Phenyl;
R' Alkyl mit 1-4 C-Atomen;
R" Methyl, Methoxy, Cyano oder Halogen bedeuten;
mit der Massgabe, dass R¹-Z³ keine -O-O- oder -N-O- Gruppen enthalten.

3. Verbindungen gemäss Anspruch 1 oder 2, worin die mesogenen Reste A¹ und A² je einen Rest der allgemeinen Formel II 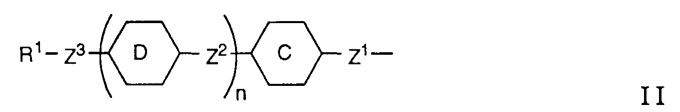 bedeuten, worin
worin
Ringe C und D unabhängig voneinander Pyridin-2,5-diyl, Pyrimidin-2,5-diyl, trans-1,4-Cyclohexylen, trans-1,3-Dioxan-2,5-diyl oder gegebenenfalls mit Halogen, Methyl und/oder Cyano substituiertes 1,4-Phenylen;
Z¹ -CH₂-, -CO-, -(CH₂)₃- oder -(CH₂)₂CO-;
Z² eine Einfachbindung, -CH₂CH₂-, -CH₂O-, -OCH₂-, -COO-, -OOC-, -(CH₂)₄-, -O(CH₂)₃- oder -(CH₂)₃O-;
Z³ -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, oder -NHCH₂(Si[(CH₃)₂]O)ₘSi[(CH₃)₂]CH₂NH-;
Y Wasserstoff oder Fluor;
n 0 oder 1;
m eine ganze Zahl von 1 bis 16; und
R¹ vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 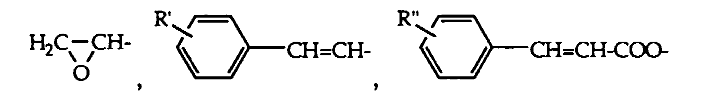
Ph Phenyl;
R' Alkyl mit 1-4 C-Atomen;
R" Methyl, Methoxy, Cyano oder Halogen bedeuten;
mit der Massgabe, dass R¹-Z³ keine -O-O- oder -N-O- Gruppen enthalten.

4. Verbindungen gemäss Anspruch 2 oder 3, worin die mesogenen Reste A¹ und A2 die gleiche Bedeutung haben.

5. Verbindungen gemäss einem der Ansprüche 1 bis 4, worin die Reste A¹ und A² einen Rest der Formel II-a 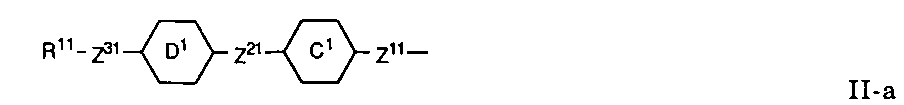 bedeuten, worin
Ringe C¹ und D¹ unabhängig voneinander Pyridin-2,5-diyl, Pyrimidin-2,5-diyl oder gegebenenfalls mit Fluor substituiertes 1,4-Phenylen, trans-1,4-Cyclohexylen;
Z¹¹ -CH₂- oder -CO-;
Z²¹ eine Einfachbindung, -CH₂O-, -COO-, -OOC-;
Z³¹ -(CH₂)_{m'}-, -(CH₂)_{m'}O-, -(CH₂)_{m'}COO- oder -(CH₂)_{m'}OOC-;
m' eine ganze Zahl von 3 bis 12; und
R¹¹ CH₂=CH-COO-, CH₂=C(CH₃)-COO-, CH₂=CH-O-, 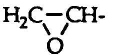
bedeuten.

6. Verbindungen gemäss Anspruch 5 der Formeln IA-IE 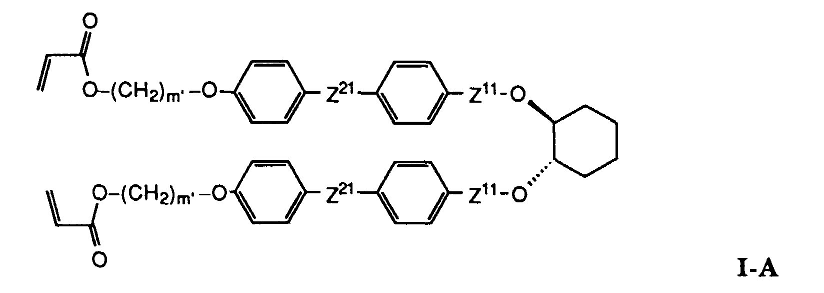 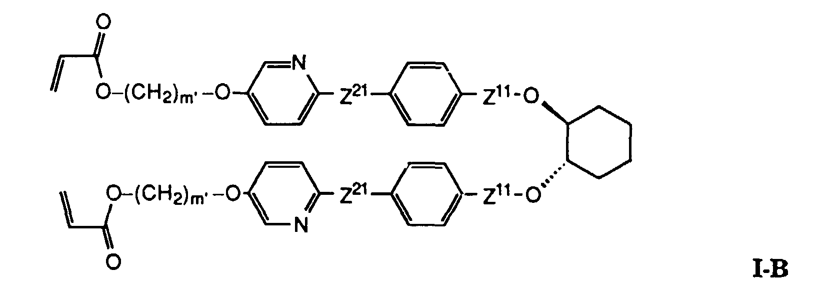 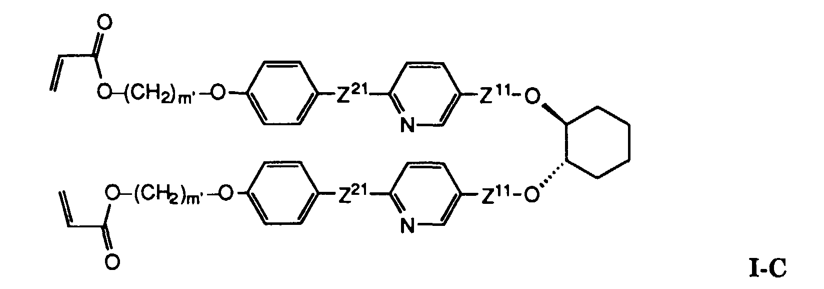 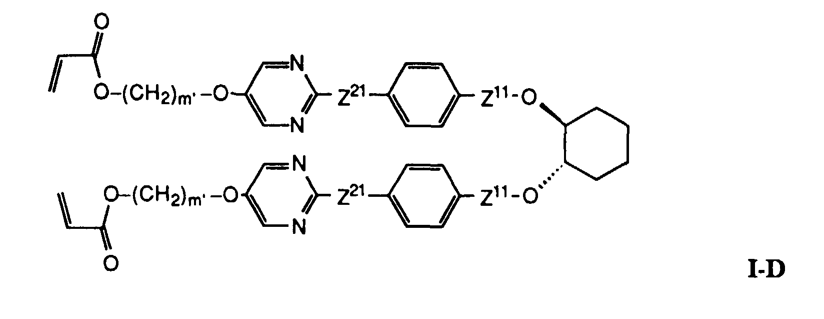 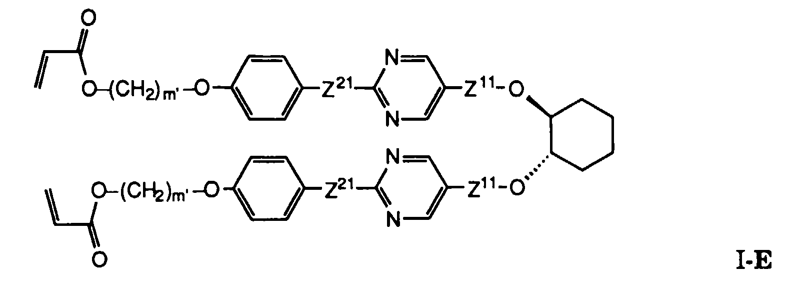 worin Z¹¹ -CO- und Z²¹ eine Einfachbindung oder -COO-, insbesondere jedoch -COO-, bedeuten, und m' eine ganze Zahl von 3 bis 12 ist, sowie die Enantiomeren dieser Verbindungen.

7. Vernetzbare, flüssigkristalline Gemische bestehend aus mindestens 2 Komponenten, wovon mindestens eine Komponente eine optisch aktive Verbindung der in Anspruch 1 definierten Formel I ist.

8. Vernetzbare, flüssigkristalline Gemische gemäss Anspruch 7, dadurch gekennzeichnet, dass sie neben einer oder mehreren Verbindungen der Formel I, eine oder mehrere Verbindungen aus der Gruppe der Formeln 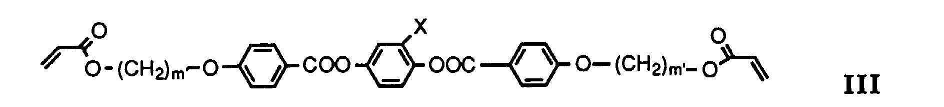 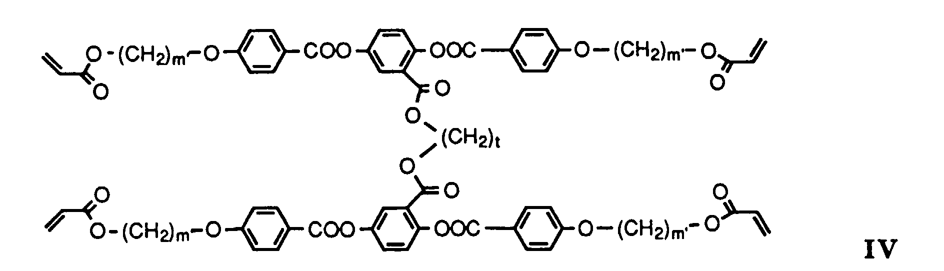 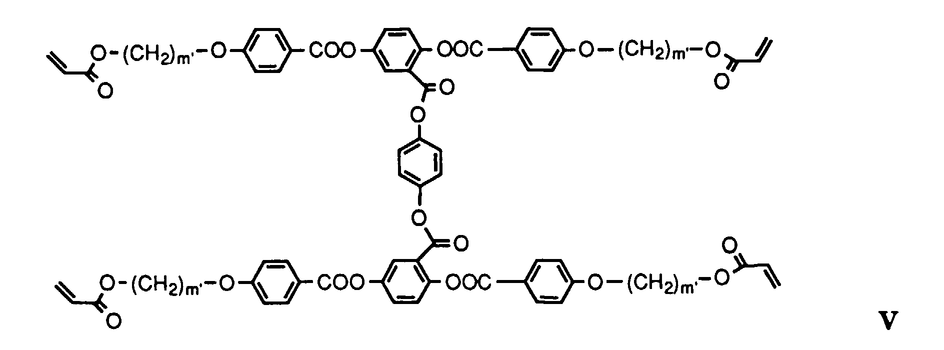 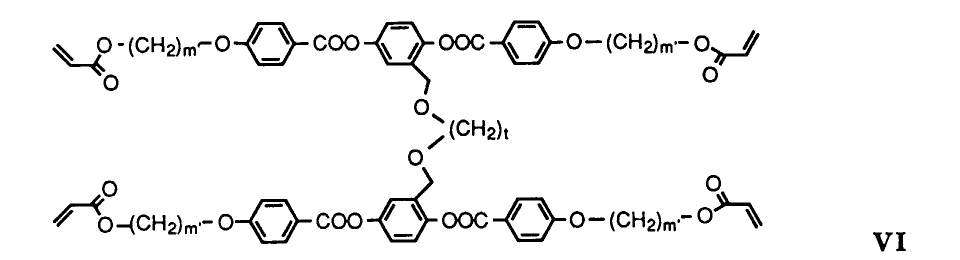 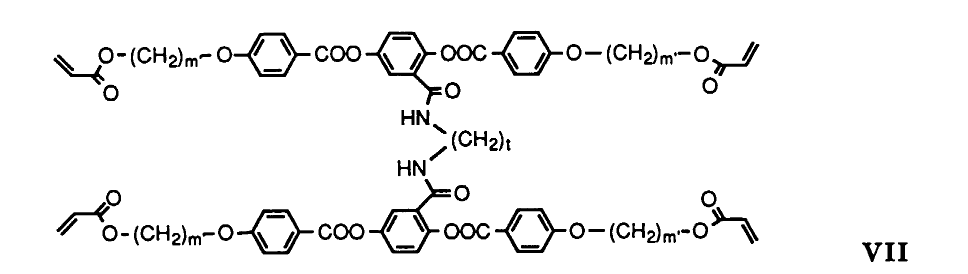 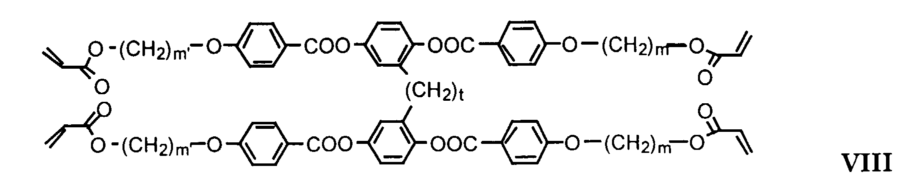 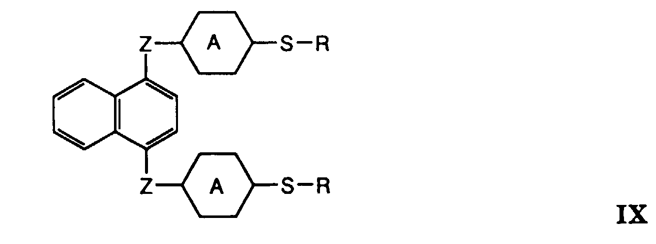 und 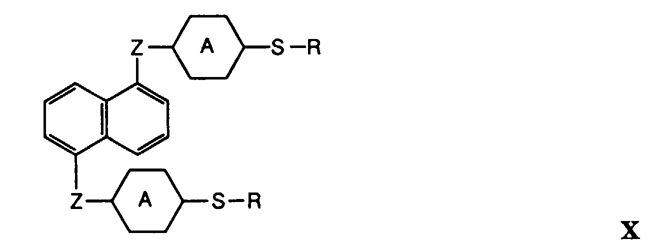 worin
X Wasserstoff, Fluor, Chlor, Brom oder Methyl;
m' eine ganze Zahl von 3 bis 12;
t eine ganze Zahl von 2 bis 12 ist;
Z -OCH₂- oder -OOC-;
A 1,4-Phenylen oder 2- bzw. 3-Fluor-1,4-phenylen;
S -(CH₂)_{m'}- oder -(CH₂)_{m'}O- oder -O-(CH₂)_{m'}-
R Acrylat, Methacrylat, Vinylether oder Epoxy bedeuten.

9. Verwendung von Verbindungen gemäss einem der Ansprüche 1 bis 6 in ihrem vernetzten Zustand für optische Bauelemente.

10. Verwendung von vernetzbaren flüssigkristallinen Gemischen gemäss einem der Ansprüche 7 oder 8 in ihrem vernetzten Zustand für optische Bauelemente.

## Claims

1. Compounds of the general formula I 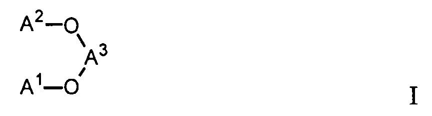 wherein
A¹ and A² each signify a cross-linkable mesogenic residue; and
A³ signifies (R,R)- or (S,S)-trans-1,2-cyclohexyl-diyl.

2. Compounds according to claim 1, wherein the mesogenic residues A¹ and A² each signify a residue of general formula II 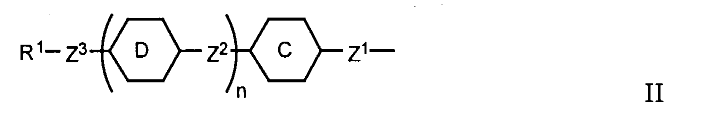 wherein
rings C and D each independently signify pyridine-2,5-diyl, pyrimidine-2,5-diyl, trans-1,4-cyclohexylene, trans-1,3-dioxane-2,5-diyl or 1,4-phenylene optionally substituted with halogen, methyl and/or cyano;
Z¹ signifies -(CH₂)ₘ-, -CO-, -(CH₂)ₘCO- or -(CH₂)ₘOOC-;
Z² signifies a single bond, -CH₂CH₂-, -CH₂O-, -OCH₂-, -COO-, -OOC-, -(CH₂)₄-, -O(CH₂)₃- or -(CH₂)₃O-;
Z³ signifies -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O- or NHCH₂(Si[(CH₃)₂]O)ₘSi[(CH₃)₂]CH₂NH-;
Y signifies hydrogen or fluorine;
n signifies 0, 1 or 2;
m signifies a whole number of 1 to 16; and
R¹ signifies a cross-linkable group of the structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 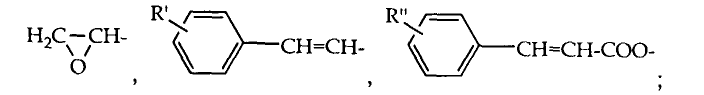
Ph signifies phenyl;
R' signifies alkyl containing from 1 to 4 carbon atoms; and
R" signifies methyl, methoxy, cyano or halogen;
with the proviso that R¹-Z³ contains no -O-O- or -N-O- groups.

3. Compounds according to claim 1 or 2, wherein the mesogenic residues A¹ and A² each signify a residue of general formula II 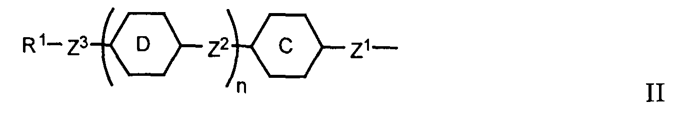 wherein
rings C and D each independently signify pyridine-2,5-diyl, pyrimidine-2,5-diyl, trans-1,4-cyclohexylene, trans-1,3-dioxane-2,5-diyl or 1,4-phenylene optionally substituted with halogen, methyl and/or cyano;
Z¹ signifies -CH₂-, -CO-, -(CH₂)₃- or -(CH₂)₂CO-;
Z² signifies a single bond, -CH₂CH₂-, -CH₂O-, -OCH₂-, -COO-, -OOC-, -(CH₂)₄-, -O(CH₂)₃- or -(CH₂)₃O-;
Z³ signifies -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O- or -NHCH₂(Si[(CH₃)₂]O)ₘSi[(CH₃)₂]CH₂NH-;
Y signifies hydrogen or fluorine;
n signifies 0 or 1;
m signifies a whole number of 1 to 16; and
R¹ signifies a cross-linkable group of the structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 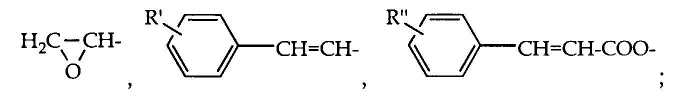
Ph signifies phenyl;
R' signifies alkyl containing from 1 to 4 carbon atoms; and
R" signifies methyl, methoxy, cyano or halogen;
with the proviso that R¹-Z³ contains no -O-O- or -N-O- groups.

4. Compounds according to claim 2 or 3, wherein the mesogenic residues A¹ and A² have the same significance.

5. Compounds according to any one of claims 1 to 4, wherein the residues A¹ and A² signify a residue of formula II-a 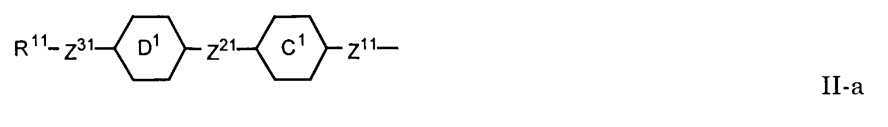 wherein
rings C¹ and D¹ each independently signify pyridine-2,5-diyl, pyrimidine-2,5-diyl, trans-1,4-cyclohexylene or 1,4-phenylene optionally substituted with fluorine;
Z¹¹ signifies -CH₂- or -CO-;
Z²¹ signifies a single bond, -CH₂O-, -COO- or -OOC-;
Z³¹ signifies -(CH₂)ₘ-, -(CH₂)_{m'}O-, -(CH₂)ₘ,COO- or -(CH₂)ₘ,OOC-;
m' signifies a whole number of 3 to 12; and
R¹¹ signifies CH₂=CH-COO-, CH₂=C(CH₃)-COO-, CH₂=CH-O- or

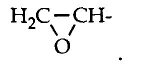

6. Compounds according to claim 5 of formulae IA-IE 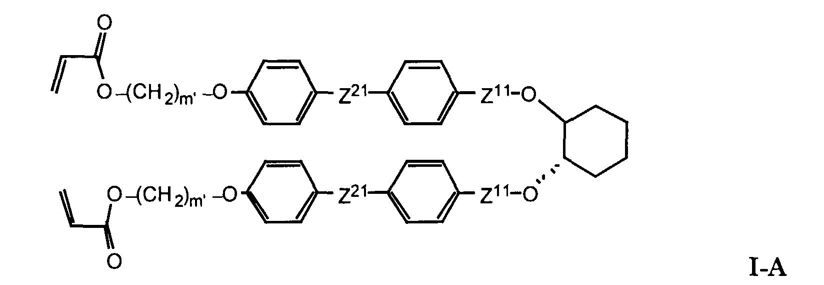 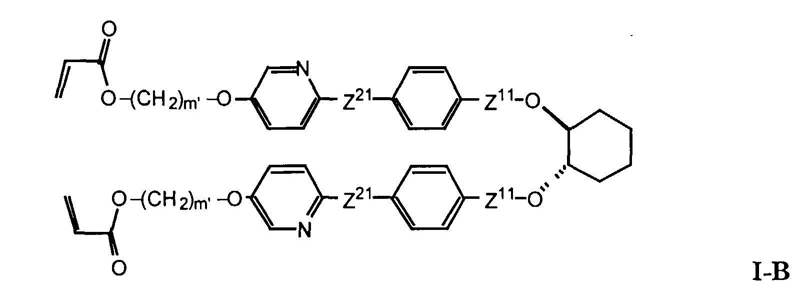 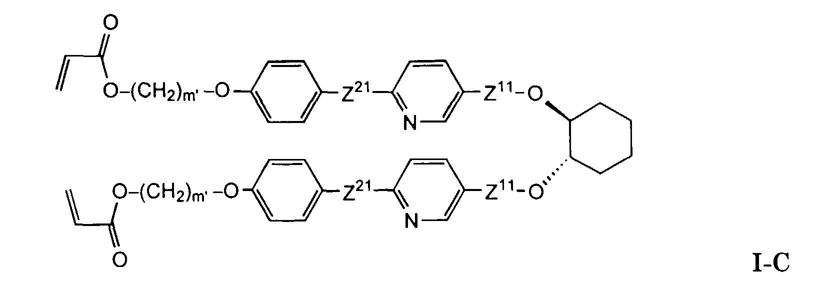 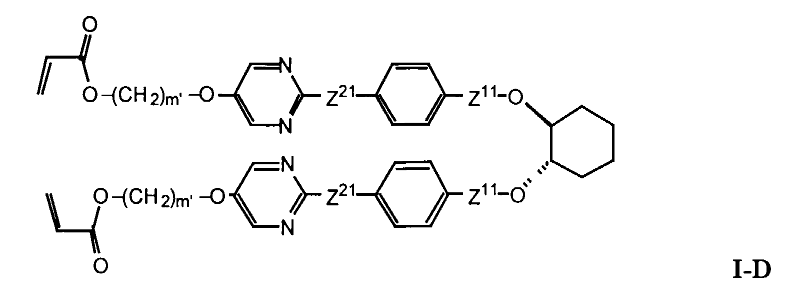 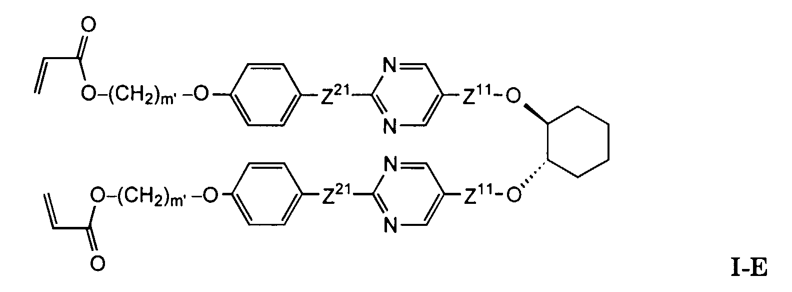 wherein Z¹¹ signifies -CO- and Z²¹ signifies a single bond or -COO-, but especially -COO-, and m' is a whole number of 3 to 12, as well as the enantiomers of these compounds.

7. Cross-linkable liquid crystalline mixtures consisting of at least 2 components, wherein at least one component is an optically active compound of the formula I defined in claim 1.

8. Cross-linkable liquid crystalline mixtures according to claim 7, characterised in that they comprise, in addition to one or more compounds of formula I, one or more compounds from the group of the formulae 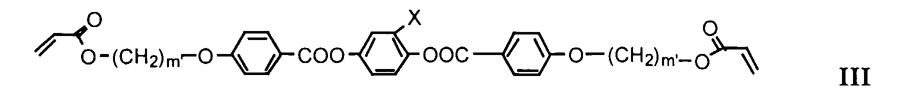 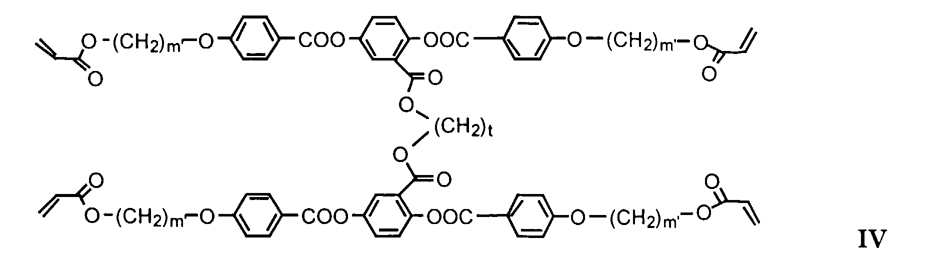 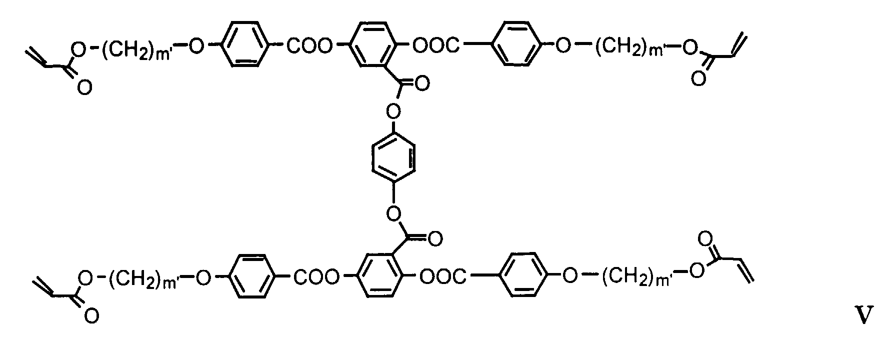 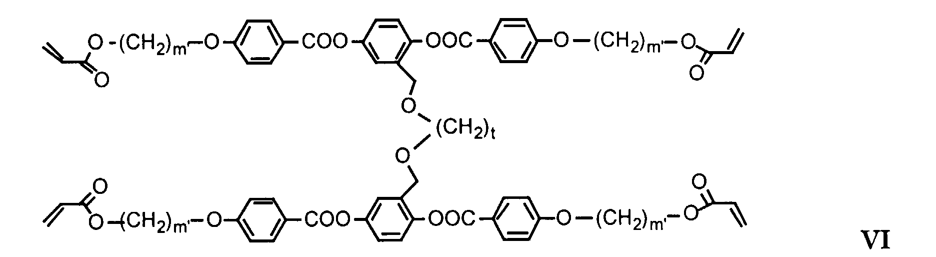 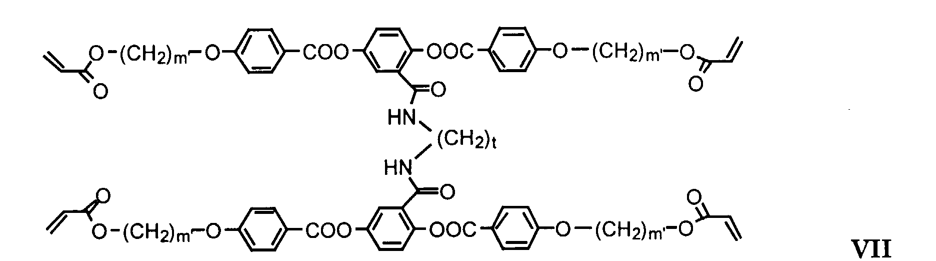 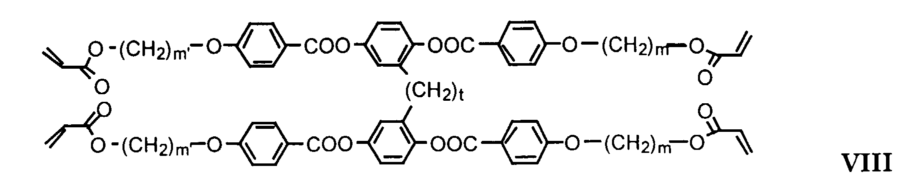 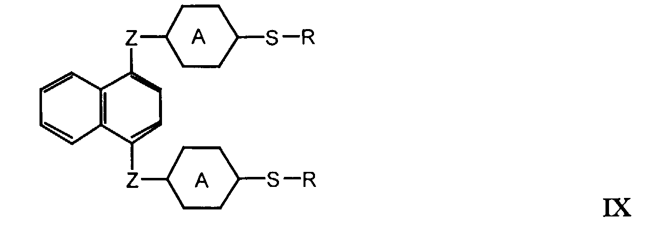 and 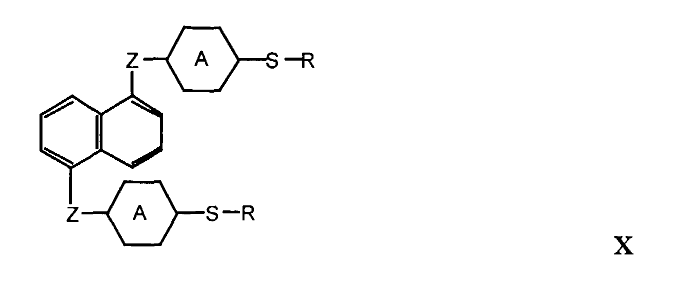 wherein
X signifies hydrogen, fluorine, chlorine, bromine or methyl;
m' signifies a whole number of 3 to 12;
t is a whole number of 2 to 12;
Z signifies -OCH₂- or -OOC-;
A signifies 1,4-phenylene or 2- or 3-fluoro-1,4-phenylene;
S signifies -(CH₂)_{m'}- or -(CH₂)ₘ,O- or -O-(CH₂)ₘ,-; and
R signifies acrylate, methacrylate, vinyl ether or epoxy.

9. The use of compounds according to any one of claims 1 to 6 in their cross-linked state for optical components.

10. The use of cross-linkable liquid crystalline mixtures according to claim 7 or 8 in their cross-linked state for optical components.

## Revendications

1. Composé de formule générale I 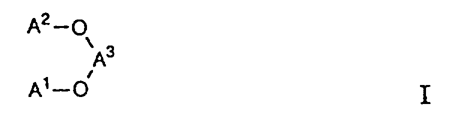 dans laquelle
A¹ et A² représentent chacun un groupe mésogène réticulable; et
A³ représente le (R,R)- ou (S,S)-trans-1,2-cyclohexyldiyle.

2. Composé selon la revendication 1, dans lequel les groupes mésogènes A¹ et A² représentent chacun un groupe de formule générale II 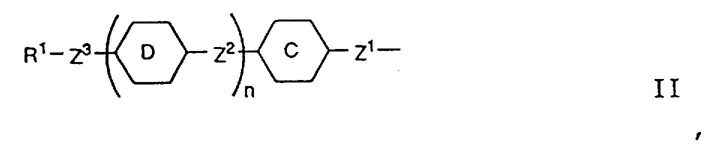 dans laquelle
les cycles C et D représentent indépendamment l'un de l'autre un groupe pyridin-2,5-diyle, pyrimidin-2,5-diyle, trans-1,4-cyclohexylène ou trans-1,3-dioxan-2,5-diyle, ou un groupe 1,4-phénylène éventuellement substitué par un atome d'halogène, un groupe méthyle et/ou cyano;
Z¹ représente un groupe -(CH₂)ₘ-, -CO-, -(CH₂)ₘCO-, -(CH₂)ₘOOC-;
Z² représente une simple liaison, un groupe -CH₂CH₂-, -CH₂O-, -OCH₂-, -COO-, -OOC-, -(CH₂)₄-, -O(CH₂)₃- ou -(CH₂)₃O-;
Z³ représente un groupe -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘsi[(CH₃)₂]CH₂O-, ou -NHCH₂(Si[(CH₃)₂]O)ₘSi[(CH₃)₂]CH₂NH-;
Y représente un atome d'hydrogène ou de fluor;
n représente 0, 1 ou 2;
m représente un nombre entier allant de 1 à 16; et
R¹ représente des groupes réticulables de structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 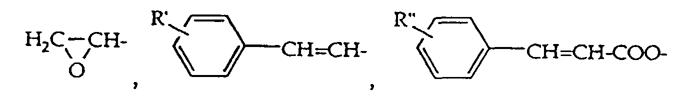
Ph représente le groupe phényle;
R' représente un groupe alkyle ayant de 1 à 4 atomes de carbone;
R" représente un groupe méthyle, méthoxy, cyano ou un atome d'halogène;
avec la condition que R¹-Z³ ne contienne pas de groupes -O-O- ni -N-O-.

3. Composé selon la revendication 1 ou 2, dans lequel les groupes mésogènes A¹ et A² représentent chacun un groupe de formule générale II 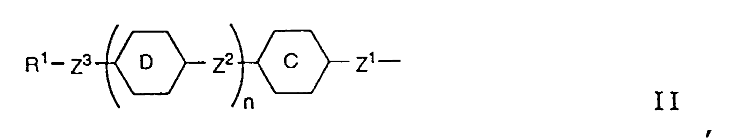 dans laquelle
les cycles C et D représentent indépendamment l'un de l'autre un groupe pyridin-2,5-diyle, pyrimidin-2,5-diyle, trans-1,4-cyclohexylène ou trans-1,3-dioxan-2,5-diyle, ou un groupe 1,4-phénylène éventuellement substitué par un atome d'halogène, un groupe méthyle et/ou cyano;
Z¹ représente un groupe -CH₂-, -CO-, -(CH₂)₃- ou -(CH₂)₂CO-;
Z² représente une simple liaison, un groupe -CH₂CH₂-, -CH₂O-, -OCH₂-, -COO-, -OOC-, -(CH₂)₄-, -O(CH₂)₃- ou -(CH₂)₃O-;
Z³ représente un groupe -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, ou -NHCH₂(Si[(CH₃)₂]O)ₘSi[(CH₃)₂]CH₂NH-;
Y représente un atome d'hydrogène ou de fluor;
n représente 0, ou 1;
m représente un nombre entier allant de 1 à 16; et
R¹ représente des groupes réticulables de structure CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 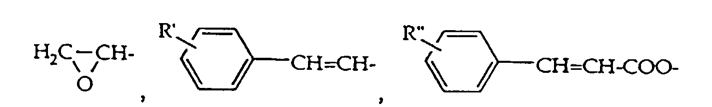
Ph représente le groupe phényle;
R' représente un groupe alkyle ayant de 1 à 4 atomes de carbone;
R" représente un groupe méthyle, méthoxy, cyano ou un atome d'halogène;
avec la condition que R¹-Z³ ne contienne pas de groupes -O-O- ni -N-O-.

4. Composé selon la revendication 2 ou 3, dans lequel les groupes mésogènes A¹ et A² ont la même signification.

5. Composé selon l'une des revendications 1 à 4, dans lequel les groupes A¹ et A² représentent un groupe de formule II-a 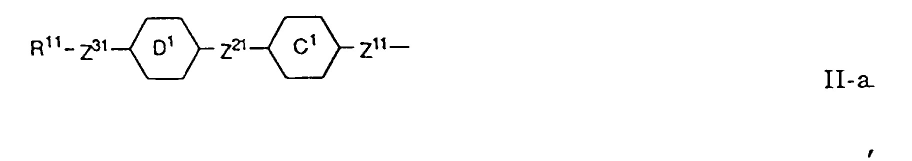 dans laquelle
les cycles C¹ et D¹ représentent indépendamment l'un de l'autre un groupe pyridin-2,5-diyle, pyrimidin-2,5-diyle ou 1,4-phénylène éventuellement substitué avec du fluor, trans-1,4-cyclohexylène;
Z¹¹ représente un groupe -CH₂ ou -CO-;
Z²¹ représente une simple liaison, un groupe -CH₂O-, -COO-, -OOC-;
Z³¹ représente un groupe -(CH₂)ₘ,-, -(CH₂)_{m'}O-, -(CH₂)_{m'}COO- ou -(CH₂)_{m'}OOC- ;
m' représente un nombre entier allant de 3 à 12; et
R¹¹ représente un groupe CH₂=CH-COO-, CH₂=C(CH₃)-COO-, CH₂=CH-O-,

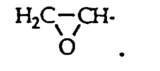

6. Composés selon la revendication 5 de formules I-A à I-E 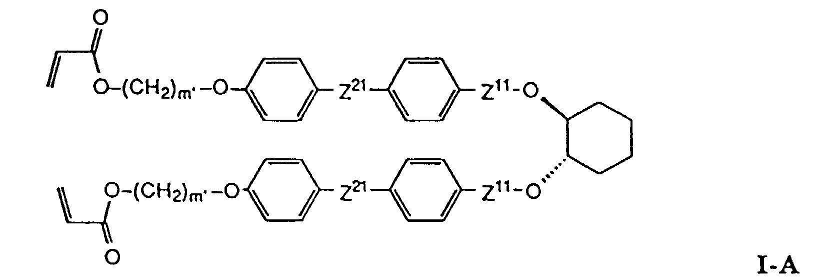 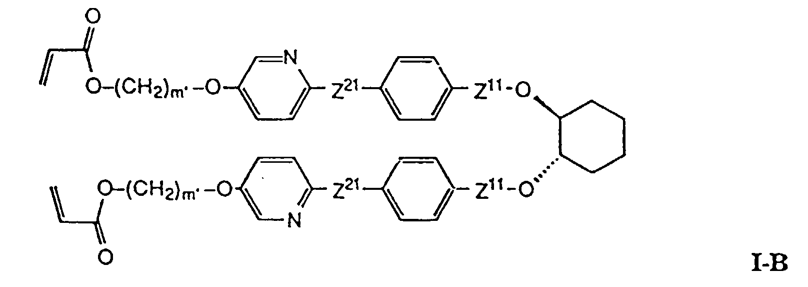 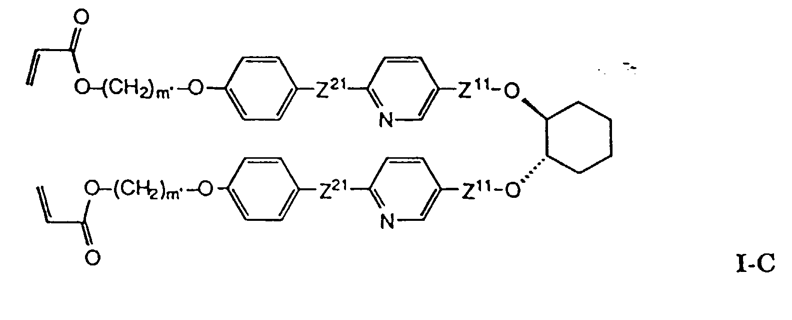 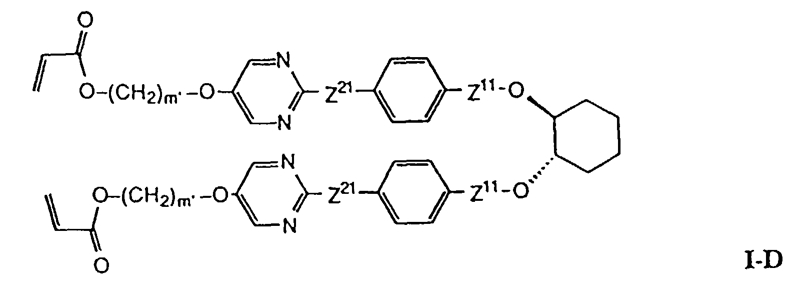 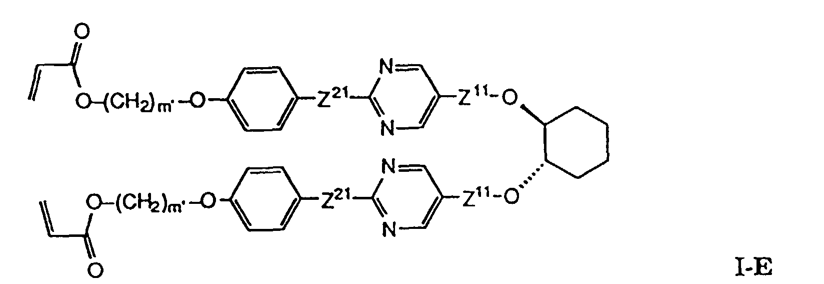 dans lesquelles Z¹¹ représente -CO- et Z²¹ représente une simple liaison ou -COO-, en particulier cependant -COO-, et m' est un nombre entier allant de 3 à 12, ainsi que les énantiomères de ces composés.

7. Mélange réticulable pour cristaux liquides constitué d'au moins 2 composants, parmi lesquels au moins un composant est un composé optiquement actif de la formule I définie à la revendication 1.

8. Mélange réticulable pour cristaux liquides selon la revendication 7, caractérisée en ce qu'il contient, en plus d'un ou de plusieurs composés de formule I, un ou plusieurs composés choisis dans le groupe de formules 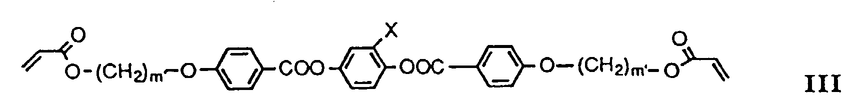 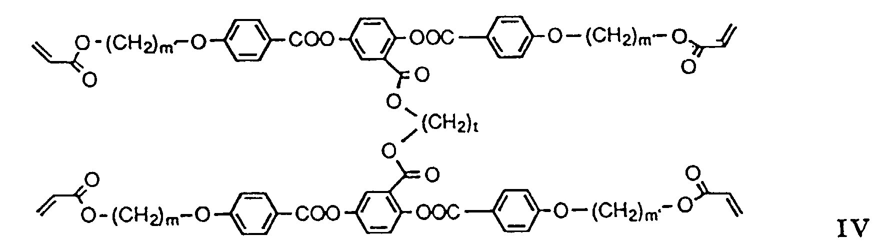 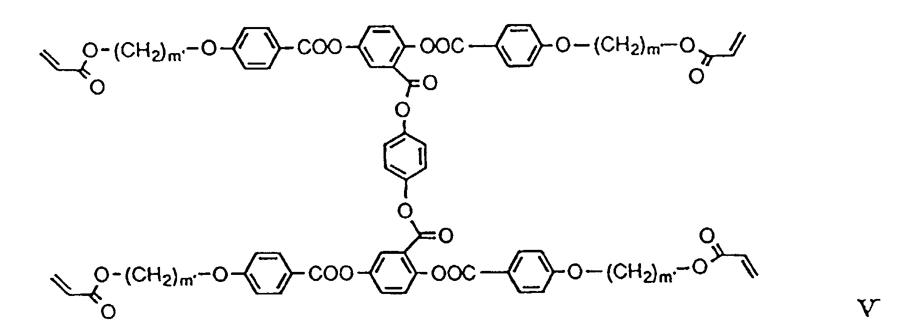 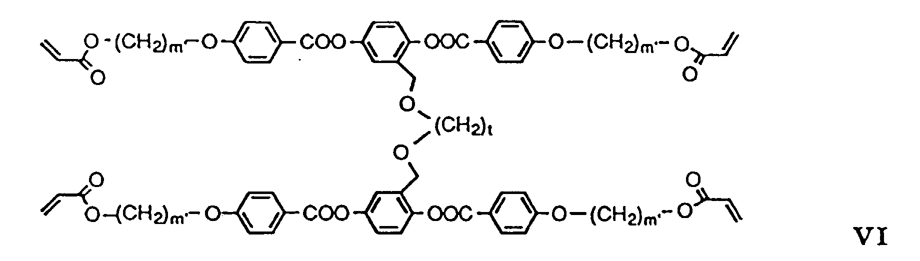 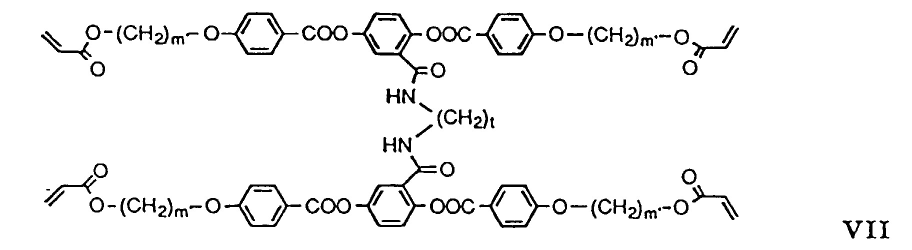 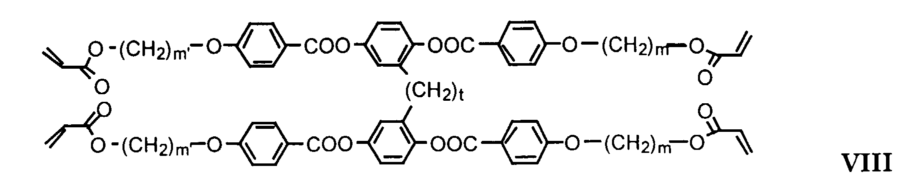 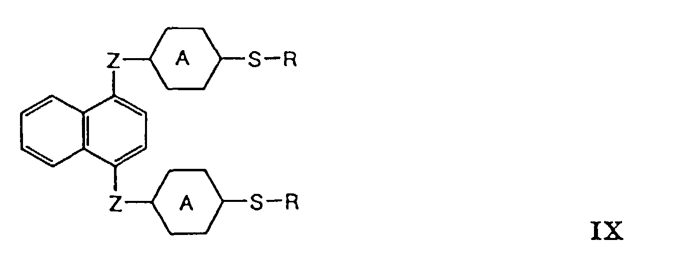 et 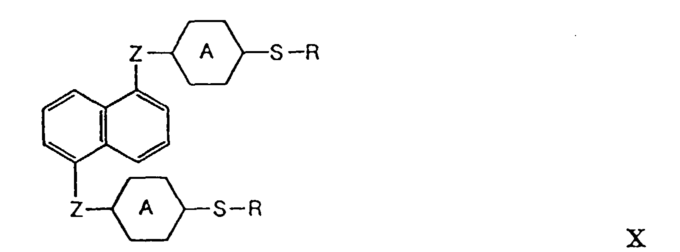 dans lesquelles
X représente un atome d'hydrogène, de fluor, de chlore, de brome ou un groupe méthyle;
m' représente un nombre entier allant de 3 à 12;
t représente un nombre entier allant de 2 à 12;
Z représente un groupe -OCH₂- ou -OOC-;
A représente un groupe 1,4-phénylène ou 2- ou 3-fluoro-1,4-phénylène;
S représente un groupe -(CH₂)_{m'} ou -(CH₂)_{m'}O- ou -O-(CH₂)_{m';}
R représente un groupe acrylate, méthacrylate, vinyléther ou époxy.

9. Utilisation de composés selon l'une des revendications 1 à 6 dans leur état réticulé pour des composants optiques.

10. Utilisation de mélanges pour cristaux liquides réticulés selon l'une des revendications 7 ou 8 dans leur état réticulé pour des composants optiques.
